# EUROPEAN PATENT APPLICATION

(11) **EP 3 943 944 A1**
(43) Date of publication of application: **26.01.2022**
(21) Application number: 20382667.2
(22) Date of filing: 24.07.2020
(51) Int. Cl.: G01N 33/569

(54) **FLOW CYTOMETRY MULTIPLEXED METHOD FOR THE DETECTION OF SARS-COV-2 ANTIBODIES**

(71) Applicant: Consejo Superior de Investigaciones Científicas (CSIC), 28006 Madrid (ES)
(72) Inventor: ALARCÓN SÁNCHEZ, Balbino, 28049 Madrid (ES); HORNDLER GIL, Lydia, 28049 Madrid (ES); DELGADO CAÑAVERAS, Pilar, 28049 Madrid (ES); BALABANOV, Ivaylo, 28049 Madrid (ES); MARTIEN VAN SANTEN, Hisse, 28049 Madrid (ES)
(74) Representative: Pons

(57) **Abstract**

The present invention relates to a first method for *in vitro* detection of anti-S protein antibodies against SARS-CoV2 in a subject and a second method for diagnostic of the exposure to SARS-CoV2 virus of a subject, based both in flow cytometry, which comprising stable expressing either by transfection or viral transduction of a vector comprising the S protein of SARS-CoV2 in a cell line in suspension. Further, the present invention relates to a kit for *in vitro* detection of anti-S protein antibodies against SARS-CoV2 and a kit for diagnostic of the exposure to SARS-CoV2, as well as its uses.

## Description

The invention relates to detection of SARS CoV-2 antibodies, more particularly, to a method for *in vitro* detection of anti-S protein antibodies against SARS-CoV2 and a second method for diagnostic of the exposure to SARS-CoV2 virus of a subject, based both in flow cytometry.

### BACKGROUND ART

Severe Acute Respiratory Syndrome Coronavirus-2 (SARS-CoV-2), is the causative agent of the global pandemic COVID-19. Phylogenetic analysis of the full genome classifies SARS-CoV-2 as a *Betacoronavirus subgenus Sarbecovirus,* lineage B and is related to bat isolates of SARS-CoV and is 79% identical to the SARS virus causing a viral epidemic in 2002.

As other coronaviruses, SARS-CoV-2 encodes for 16 non-structural proteins at the 5' end of the genomic RNA and structural proteins spike (S), envelope (E), membrane (M) and nucleocapsid (N) at the 3' end. The spike S protein is responsible for binding to ACE2 in host cells which seems to be the main cellular receptor for the virus (Hoffmann, M., et al, 2020, Cell 181, 271-280*.e8*). In native state, the S protein forms homotrimers and is composed of two fragments S1 and S2 that result from proteolytic cleavage of S. The S1 fragment contains a central sequence RBD that is actually the ACE2-binding sequence and the target of neutralizing antibodies such as those described to neutralize SARS-CoV-1 (Walls, A. C., etal, 2020, Cell 181, 281-292.e6).

Diagnosis of active infection is currently carried out by PCR amplification of viral RNA comprising fragments of the N, E, S and RdRP genes from biological samples taken from bronchoalveolar lavage (BAL), sputum, nasal swabs, pharyngeal swabs and fibronchoscope brush biopsies, with the highest positive rate resulting from PCR of BAL samples. Positivity in the PCR test vanishes as the infection is resolved although viral RNA shedding, and PCR positivity, could be taken place even when there is no longer production of infective virions and therefore possibility of transmission.

Unlike PCR-based tests, serological tests are not highly valuable to determine what individual has an active infection with SARS-CoV-2 but can provide an estimate of what segment of a population has been infected with the virus and is likely to have acquired total or partial immunity against possible resurgences of the pandemics. Herd immunity achieved either by natural infection or as a consequence of vaccination is a goal for all health authorities in the world.

Seropositivity is usually established by detecting the presence of IgG or IgM in the serum of individuals using recombinant fragments of the S or N proteins and tests based on ELISA or lateral flow assays (Venter, M. & Richter, K., 2020, J. Clin. Pathol. 73, 370-377). A disadvantage of those tests is that neutralizing antibodies are not directed against the N protein and that recombinant fragments of S miss the quaternary structure of the S protein trimer which is the native form of the spike protein in the viral envelope. Therefore, possible neutralizing antibodies directed against the native S trimer could be missed in serological tests based on the expression of recombinant proteins.

An alternative strategy for a serological test is necessary to detect antibodies against the SARS-CoV-2.

### SUMMARY OF THE INVENTION

The present invention discloses a method for detection of antibodies against SARS-CoV2 based on flow cytometry of cells, which are transfected with vectors that express the S protein. So, the detection of antibodies against the S protein of SARS-CoV2 allows to diagnose whether a subject has been exposed to the SARS-CoV2.

The inventors have developed a test based in flow cytometry for detection of anti-S protein antibodies against SARS-CoV2, using stably transfected Jurkat human hematopoietic cells in suspension that co-express the native S protein of SARS-CoV-2 (SEQ ID NO:1) and a truncated form of the human EGFR (SEQ ID NO:2) that serves as a normalizer. Further the inventors have observed that the method based in flow cytometry is superior to ELISA-based methods to detect sera of donors containing neutralizing antibodies. In comparison with commercial serological tests, the Jurkat-S clone-based flow cytometry (FC) test can detect more cases of seropositive individuals (Table 1).

So, a first aspect of the present invention related to a method for *in vitro* detection of anti-S protein antibodies against Severe Acute Respiratory Syndrome Coronavirus-2 (SARS-CoV-2) by flow cytometry in a subject, hereinafter the "method of the invention", comprising the following steps:
(a) stable expressing either by transfection or viral transduction of a vector comprising a nucleotide sequence with at least 70% identity with the nucleotide sequence SEQ ID NO: 1 in a cell line in suspension,
(b) incubating the cell line obtained in step (a) with a sample isolated from the subject,
(c) incubating the sample obtained in step (b) with fluorescence labelled secondary antibodies, and
(d) measuring the mean fluorescence intensity of the sample obtained in step (c) by flow cytometry for detecting anti-S protein antibodies,
wherein mean fluorescence intensity is higher than a reference value is considered positive for anti-S protein antibodies.

The term "anti-S protein antibody" or "anti-S protein antibodies" as used in the method of the invention, relates to anti-S proteins, i.e. immunoglobulin molecules and immunologically active portions (or fragments) of immunoglobulin molecules against the S protein of SARS-CoV2. That is, it refers to molecules that specifically bind (are immunoreactive) to an antigen, such as, for example, a peptide or a protein (an immunogen or epitope). In the present invention the method relates the *in vitro* detection of antibodies against SARS-CoV2 or anti-S protein antibodies, i.e., molecules that specifically bind with S protein of SARS-CoV2. As is known by a person skilled in the art, there are five isotypes or main classes of immunoglobulins: immunoglobulin M (IgM), immunoglobulin D (IgD), immunoglobulin G (IgG) (which in turn have the following subtypes: IgG1, IgG2, IgG3 and IgG4), immunoglobulin A (IgA) and immunoglobulin E (IgE).

For detecting the antibodies against SARS-CoV2 in the method of the invention, the inventors have stable expressed either by transfection or viral transduction of a vector comprising a nucleotide sequence with at least 70% identity with the nucleotide sequence of the S protein of SARS-CoV2 (SEQ ID NO: 1) in a cell line in suspension.

The term "stable expression" as used in the present invention, relates to generation or process of homogenous populations of cells that demonstrate expression of a transfected gene insert and the transfected gene integrates into the genome of the host cell, as a result, are able to express the transfected genetic material.

In the present invention the cell line, where is integrated the vector that comprising a nucleotide sequence with at least 70% identity with the nucleotide sequence of the S protein of SARS-CoV2 (SEQ ID NO: 1), is a cell line in suspension. The term "cell line in suspension" used herein, refers to cell line or cell populations that are non-adhesive cell lines and can be grown floating in the culture medium without attaching to a surface. In a preferred embodiment of the method of the invention, the cell line in suspension is a mammal cell line, preferably human cell, more preferably is a non-adherent hematopoietic human cell line. Examples of non-adherent hematopoietic human cell line including, without limitation to, myeloid leukemic cell lines K562, U937 and Daudi.

In a more preferred embodiment of the method of the invention, the cell line in suspension is a lymphoblast cell line. Examples of lymphoblast cell line in the present invention include, without limitation to, T cell leukemic lines Jurkat, CEM, Molt4, SupT1, HUT78, HSB-2, PEER, B cell leukemic lines Raji, Ramos, MEC1, Sudha6 and Sudha8.

In an even more preferred embodiment of the method of the invention, the cell line in suspension is the human T cell leukemic line Jurkat.

In the present invention, the cell line in suspension is transfected or viral transduced with a vector comprising the S protein of SARS-CoV2 (SEQ ID NO: 1). The "spike S protein" or "S protein", as used in the present invention, is the structural protein encoded for SARS-CoV-2, responsible for binding to ACE2 in host cells which seems to be the main cellular receptor for the virus. The S protein of SARS-CoV-2 of the method of invention comprises at least 70% identity with the nucleotide sequence SEQ ID NO: 1.

In the present invention, "identity" or "sequence identity" refers to the degree of similarity between two nucleotide sequences or the amino acids obtained by aligning the two sequences. Depending on the number of common residues between the aligned sequences, a degree of identity is expressed as a percentage. The degree of identity between two parameter sequences can be determined by standard methods, for example, by standard sequence alignment algorithms known in the state of the art, such as BLAST. The BLAST programs, for example, BLASTN, BLASTX and TBLASTX, BLASTP and TBLASTN, are in the public domain on the website of the National Center for Biotechnology Information (NCBI). In a particular embodiment, the vector of the method of invention comprises a nucleotide sequence with at least 80% identity with the nucleotide sequence SEQ ID NO: 1, preferably comprises an identity of at least 81, 82, 83, 84, 85, 86, 87, 88, 89, 90, 91, 92, 93, 94, 95, 96, 97, 98 or 99% with the sequence SEQ ID NO: 1. In another more particular embodiment, the vector of the method of invention comprises a nucleotide sequence with at least 100% identity with the nucleotide sequence SEQ ID NO: 1. In another even more particular embodiment, the vector of the method of invention comprises a nucleotide sequence consisting of the sequence SEQ ID NO: 1. As understood by the person skilled in the art, all peptides that have an identity of at least 70% with the sequence SEQ ID NO: 1 and which have the same function as SEQ ID NO: 1, they are functionally equivalent variants of S protein of SARS-CoV-2 of the method of the invention. Assays to identify proteins functionally equivalent to the S protein of SARS-CoV-2 are routine practice for the skilled in the art.

In the method of the invention, the S protein of SARS-CoV2 is inserted in a vector for stable expression either by transfection or viral transduction in the cell line in suspension.

In the present invention, the term "vector" refers to a nucleic acid molecule that can be used to transport or transfer a nucleotide sequence into the interior of a cell. Some vectors are capable of autonomously replicating or dividing after being introduced into the host cell and other vectors can be integrated into the host cell genome and thus replicate along with the cell genome. A vector may contain different functional elements including, but not limited to, transcription control elements, such as promoters or operators, regions or enhancers of transcription factor binding, and control elements for starting and ending transcription. Vectors include, but are not limited to: plasmids, cosmids, viral vectors, phages, recombinant expression cassettes, and transposons. It is routine practice for skilled in the art to determine the vector of choice depending the host cell and the use. The vector of the method of the invention can be obtained by conventional methods known to the person skilled in the art. In a preferred embodiment of the method of the invention the vector is a viral vector. Examples of vectors in the present invention include, without limitation to, lentiviral vectors containing the Long Terminal Repeats (LTRs) of human immunodeficiency virus, retroviral vectors containing the LTRs of oncogenic retroviruses, adenoviral vectors, Adeno-Associated Viral Vectors and vaccinia virus-derived vectors.

As a person skilled in the art knows, vectors can contain selection markers for transfected or transduced cells. In other preferred embodiment of the method of the invention, the vector of step (a) further comprising a marker of transfection or viral transduction. Examples of marker of transfection or viral transduction in the present invention include, without limitation to, truncated human EGFR, human CD2 and green fluorescent protein (GFP) and its variants.

In other more preferred embodiment of the method of the invention, the marker is a truncated human EGFR protein.

In other even more preferred embodiment of the method of the invention the truncated human EGFR protein comprises the nucleotide sequence SEQ ID NO: 2.

In other even more preferred embodiment of the method of the invention, the marker is a truncated human EGFR protein separated from the nucleotide sequence with at least 70% identity with the nucleotide sequence SEQ ID NO: 1 by a proteolytic T2A cleavage site.

In other even more preferred embodiment of the method of the invention the proteolytic T2A cleavage site comprises the nucleotide sequence SEQ ID NO: 3.

The methods for inserting or integrating the vector into the host cell and stable expressing the S protein of SARS-CoV2, are widely known in the state of the art. Examples of methods for inserting or integrating a vector into the host cell include, without limitation to, chemical transformation, liposomes, transfection, electroporation, particle bombardment, gene guns, microinjection and viral transduction.

In another preferred embodiment of the method of the invention, the vector is integrated into the host cell either by transfection or viral transduction.

In the present invention, the term "transfection" or "transformation" refers to process of deliberately introducing naked or purified nucleic acids into eukaryotic cells and it involves opening transient pores or holes in the cell membrane to allow the uptake of material. Transfection can be carried out using calcium phosphate (i.e. tricalcium phosphate), by electroporation, by cell squeezing or by mixing a cationic lipid with the material to produce liposomes that fuse with the cell membrane and deposit their cargo inside.

In the present invention, the term "viral transduction" refers to process by which foreign DNA is introduced or inserted into a cell by a virus or viral vector without physical contact between the cell donating the DNA and the cell receiving the DNA.

To determine if a biological sample is positive for anti-S protein antibodies, i.e. immunoglobulins anti-S, the stably transfected or transducted cells, previously incubated with a dilution of the isolated sample from the subject, are incubated with a dilution of the fluorescence labelled secondary antibody.

In the present invention, the term "secondary antibodies" refers to antibodies (or immunoglobulins) able to bind or target another (primary) antibody and provide signal detection and amplification through conjugation to proteins or chemicals. In the present invention, the secondary antibodies specifically bind to anti-S protein antibodies, i.e. antibodies against SARS-CoV2.

The secondary antibodies can comprise a detectable label. The term "detectable label" or "tackle" in the present invention refers to a molecular tag that allows for the detection, location and/or identification of a molecule to which it is bound, by means of suitable detection methods and equipment, either by spectroscopic, photochemical, biochemical, immunochemical or chemical means. Examples of detectable labels for marking compounds include, but are not limited to, radioactive isotopes, enzymatic substrates, cofactors, ligands, chemiluminescent agents, fluorophores, enzymes (for example peroxidase), receptors and combinations thereof. In a preferred embodiment of the method of the invention, the secondary antibody is a fluorescence labelled secondary antibody. Methods for marking and guiding the selection of suitable tackles for different purposes are known by a person skilled in the art.

The fluorescence labelled secondary antibodies in the present invention are specific for immunoglobulins of the species of the subject, preferably, anti-human immunoglobulin of IgG, IgA, IgM or IgE isotypes. Within anti-IgG secondary antibodies, they can be specific for total IgG or specific for any of the IgG subclasses (IgG1, IgG2, IgG3, IgG4 for human). Alternatively, the secondary antibody could be specific for the light chains of immunoglobulins, for instance, for the Kappa or Lambda chains of human immunoglobulins.

Finally, for detecting anti-S protein antibodies in the method of the invention, fluorescence is measured by flow cytometry of the sample obtained, previously described. The stained cell sample is passed in a narrow stream of buffer and illuminated with lasers of different wavelength. Photodetectors identify one-by-one the light reflected by each cell in different angles and the light emitted by the cell as a consequence of excitation of the fluorophore coupled to the secondary antibody. The values collected of all the detectors are plotted to determine the distribution of the fluorescence values in the cell population analysed. Positivity can be established in terms of percentage of cells above the threshold or according to the mean fluorescence intensity (MFI) of the total cell population. If the percentage or MFI of the cells incubated with the test sample are higher than a reference value, i.e. mean of experimental replicas of the negative controls, the sample is considered positive for antibodies to the S protein, provided that an adequate statistical test establishes the significance of the differences.

The term "reference value" used herein, refers to the mean fluorescence intensity (MFI) of cells stained either with the secondary antibody alone (i.e., not incubated with any biological sample) or incubated with a biological sample, e.g. serum, from an individual known not to have been infected with SARS-CoV-2 (e.g. if such sample was taken before the COVID-19 pandemic), which serve to fix the limit above which the cells incubated with the test biological sample are considered positive.

The detection of antibodies against SARS-CoV2 by flow cytometry allows to diagnose whether a subject has been exposed to the SARS-CoV2. Hereby, a second aspect of the present invention related to a method for diagnostic of the exposure to SARS-CoV2 virus of a subject by flow cytometry, hereinafter the second method of the invention, comprising the following steps:
(a) stable expressing either by transfection or viral transduction of a vector comprising a nucleotide sequence with at least 70% identity with the nucleotide sequence SEQ ID NO: 1 in a cell line in suspension,
(b) incubating the cell line obtained in step (a) with a sample isolated from the subject,
(c) incubating the sample obtained in step (b) with fluorescence labelled secondary antibodies, and
(d) measuring the mean fluorescence intensity of the sample obtained in step (c) by flow cytometry for detecting anti-S protein antibodies,
wherein if mean fluorescence intensity of the sample is higher than a reference value, the subject has anti-S protein antibodies, and is indicative that the subject has been exposed to SARS-CoV2 virus.

The terms "anti-S protein antibody", "stable expression in a cell line", "S protein of SARS-CoV-2", "cell line", "vector", "transfection", "viral transduction", "reference value" and "secondary antibodies" have been previously defined and are fully applicable to this second aspect of the invention, as well as all particular and preferred embodiments.

The present invention relates to a method for detection of antibodies against SARS-CoV2 in a subject based on flow cytometry. This detection of antibodies against SARS-CoV2 allows to diagnose whether a subject has been exposed to the SARS-CoV2.

In a preferred embodiment of the method of the invention or the second method of the invention the subject is a mammal, preferably a human.

It is necessary, in the method or the second method of the invention, a sample isolated from the subject. In a preferred embodiment of the method of the invention or the second method of the invention the sample is selected from list consisting of: blood or mucosal fluid.

In a more preferred embodiment of the method of the invention or the second method of the invention the sample is blood serum.

A third aspect of the present invention relates to a kit or device for *in vitro* detection of anti-S protein antibodies against SARS-CoV2 or for diagnostic of the exposure to SARS-CoV2 virus in a sample isolated from a subject, hereinafter the "kit of the invention" comprising:
(i) a cell line in suspension comprising a vector wherein the vector comprising a nucleotide sequence with at least 70% identity with the nucleotide sequence SEQ ID NO: 1,
(ii) fluorescence labelled secondary antibodies, and
(iii) means for carried out a detection by flow cytometry.

The terms "anti-S protein antibody", "S protein of SARS-CoV-2", "cell line", "vector" and "secondary antibodies" have been previously defined and are fully applicable to this aspect of the invention, as well as all particular and preferred embodiments.

Examples of means for carried out a detection by flow cytometry include, without limitation to, a special apparatus equipped with laser light and photodetectors, particularly termed in state of the art as "Flow cytometer".

As a person skilled in the art knows, vectors can contain selection markers, preferably marker of transfected or transducted cells. In other preferred embodiment of the kit of the invention, the vector of (i) further comprising a marker of transfection or viral transduction. Examples of marker of transfection or viral transduction in the present invention include, without limitation to, truncated human EGFR, human CD2, Nerve Growth Factor Receptor (NGFR), GFP o modified forms of GFP.

In other more preferred embodiment of the kit of the invention, the marker is a truncated human EGFR protein.

In other even more preferred embodiment of the kit of the invention the truncated human EGFR protein comprises the nucleotide sequence SEQ ID NO: 2.

In other even more preferred embodiment of the kit of the invention, the marker is a truncated human EGFR protein separated to the nucleotide sequence with at least 70% identity with the nucleotide sequence SEQ ID NO: 1 by a proteolytic T2A cleavage site.

In other even more preferred embodiment of the kit of the invention, the proteolytic T2A cleavage site comprises the nucleotide sequence SEQ ID NO: 3.

The kit of the present invention comprising a cell line in suspension, where the vector that comprises the S protein of SARS-CoV2 is integrated. In other preferred embodiment of the kit of the invention, the cell line in suspension is a mammal cell line, preferably human cell, more preferably is a non-adherent hematopoietic human cell line.

In a more preferred embodiment of the kit of the invention of the invention, the cell line in suspension is a lymphoblast cell line. Examples of lymphoblast cell line in the present invention include, without limitation to, T cell leukemic line Jurkat, CEM, Molt4, SupT1, HUT78, HSB-2, PEER, and to B cell leukemic lines Raji, Ramos, MEC1, Sudha6 and Sudha8.

In an even more preferred embodiment of the kit of the invention, the cell line in suspension is the human T cell leukemic line Jurkat.

Other aspect of the present invention relates to use of the kit of the invention for *in vitro* detection of antibodies against SARS-CoV2 in a sample isolated from a subject.

Another aspect of the present invention relates to use of the kit of the invention for diagnostic of the exposure to SARS-CoV2 virus of a subject.

In a preferred embodiment of any use of the kit of the invention, the subject is a mammal, preferably a human.

In other preferred embodiment of any use of the kit of the invention the sample isolated from the subject is selected from list consisting of: blood or mucosal fluid.

In a more preferred embodiment of the any use of the kit of the invention the sample is blood serum.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings are provided by way of illustration and are not intended to be limiting of the present invention.

### DESCRIPTION OF THE DRAWINGS

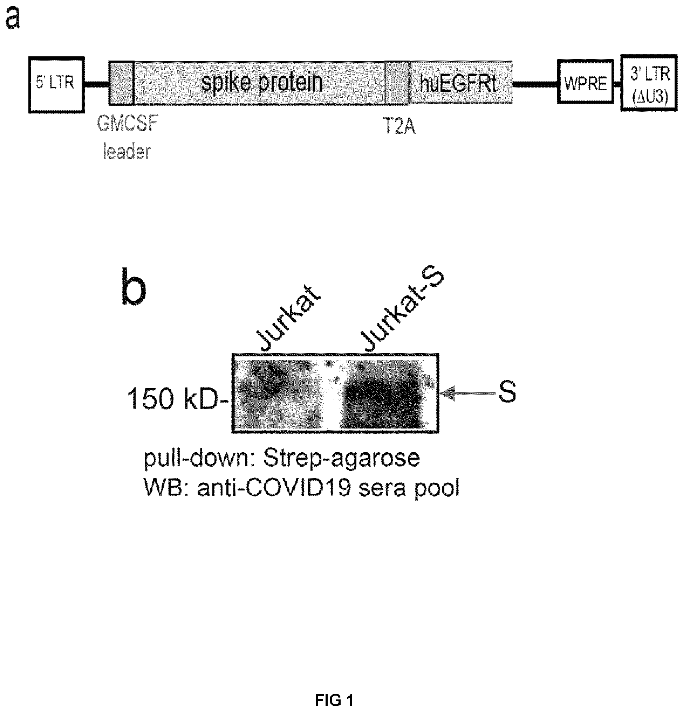

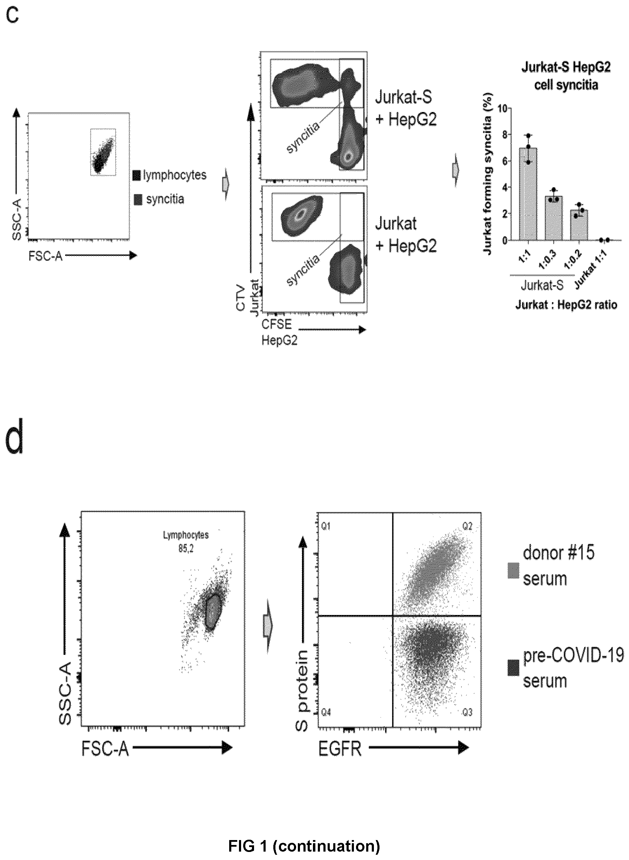

**Figure 1****.** Jurkat-S cells express native SARS-CoV-2 spike S protein allow the detection of anti-S protein antibodies in human sera. **(a)** Lentiviral construct used to permanently express S protein in Jurkat. The full-length mature S protein is preceded by a leader sequence of GM-CSF and followed by a T2A sequence which is followed by a tail-less truncated human EGFR construct. **(b)** Expression of S protein on the plasma membrane of Jurkat-S cells assessed by surface biotinylation and followed by pull-down with streptavidin-agarose, SDS-PAGE under reducing conditions and western blot with a mix of sera from donors #15, #3, #4, #8 and #66. The nitrocellulose membrane was finally blotted with a peroxidase-labeled anti-human IgG1 antibody. Biotinylation of the parental Jurkat cells was carried out in parallel as a negative control. **(c)** Formation of syncitia between CTV-labelled ACE2+ human cell line and the CFSE-labelled Jurkat-S cells was measured by flow cytometry by analyzing the percentage of cells that become double positive for CTV and CFSE markers. The Bar plot to the right shows the effect of different doses of HepG2 cells on the formation of syncitia with a fixed number of Jurkat. Parental Jurkat cells (not expressing S protein) are considered negative controls. Data represent the mean±SD of triplicated datasets. **(d)** Overlay plot of Jurkat-S cells that were incubated with anti-EGFR mAb conjugated to Bv421 and serum from either donor #15 or from a pre-COVID-19 donor and followed by a secondary anti-human lgG1 antibody conjugated to PE.

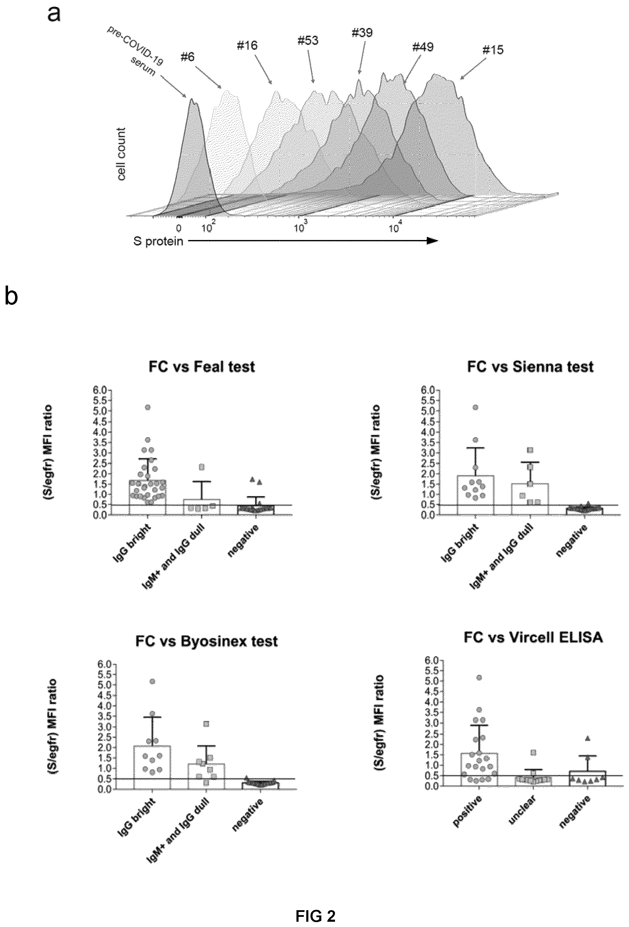

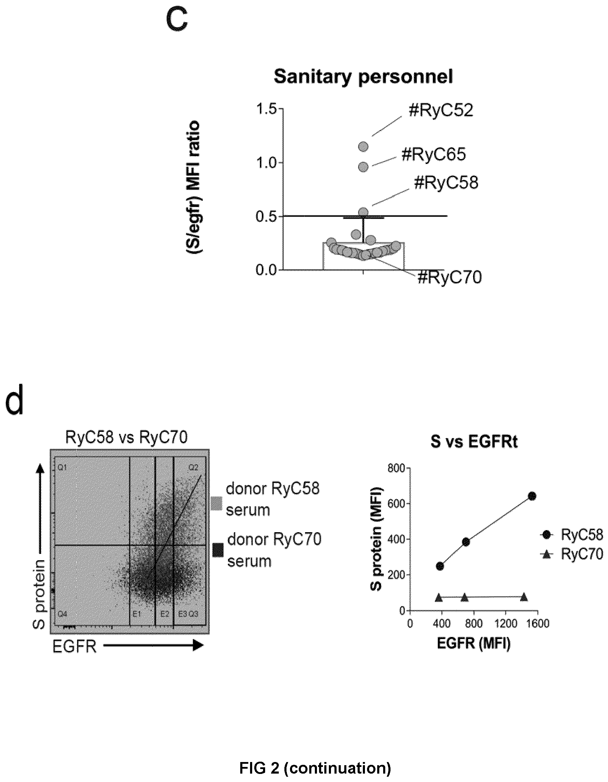

**Figure 2****.** Flow cytometry of Jurkat-S cells allows to detect anti-S immunoglobulins poorly detected by ELISA with a wide dynamic range. **(a)** Overlay histograms of Jurkat-S staining with different human sera diluted 1:50. A pre-COVID19 serum sample is taken as negative control. **(b)** Bar plots of flow cytometry data generated with Jurkat-S cells and the panel of serum samples of Table 1 classified according to their result in the indicated commercial tests: (IgG bright) positive for the test; (lgM+ and IgG dull) weak or unclear; negative samples. Flow cytometry data is expressed as the ratio between the MFI of the antibody anti-S and the MFI for EGFR. Negative values for the flow cytometry test are those with a S/EGFR MFI ratio lower than 0.5. **(c)** Bar plot showing the S/EGFR MFI ratio determined by flow cytometry analysis with Jurkat-S of serum samples from 30 sanitary personnel repeatedly tested as PCR negative for SARS-CoV-2 at the Ramon y Cajal Hospital of Madrid. A negative result is considered for a ratio lower than 0.5. Two clear cases of sera positive for anti-S lgG1 are indicated (RyC52 and RyC65). A borderline sample just above the threshold line (RyC58) is also indicated. **(d)** Two-color dot plot of anti-S protein fluorescence versus EGFR fluorescence for borderline serum sample RyC58 and the clearly negative RyC70 sample. The line plot to the right shows the MFI for EGFR taken at the 3 sectors indicated in the two plot and the corresponding MFI values for EGFR.

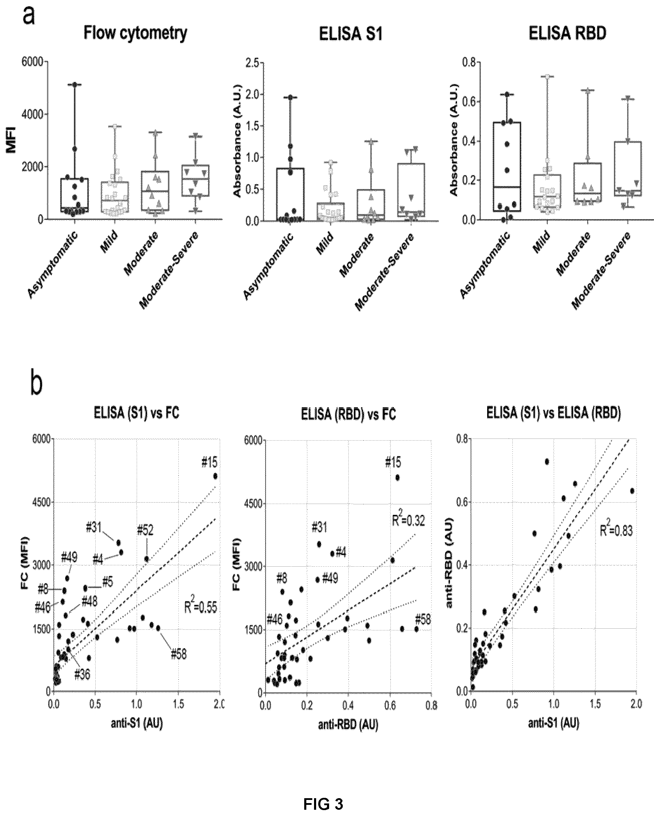

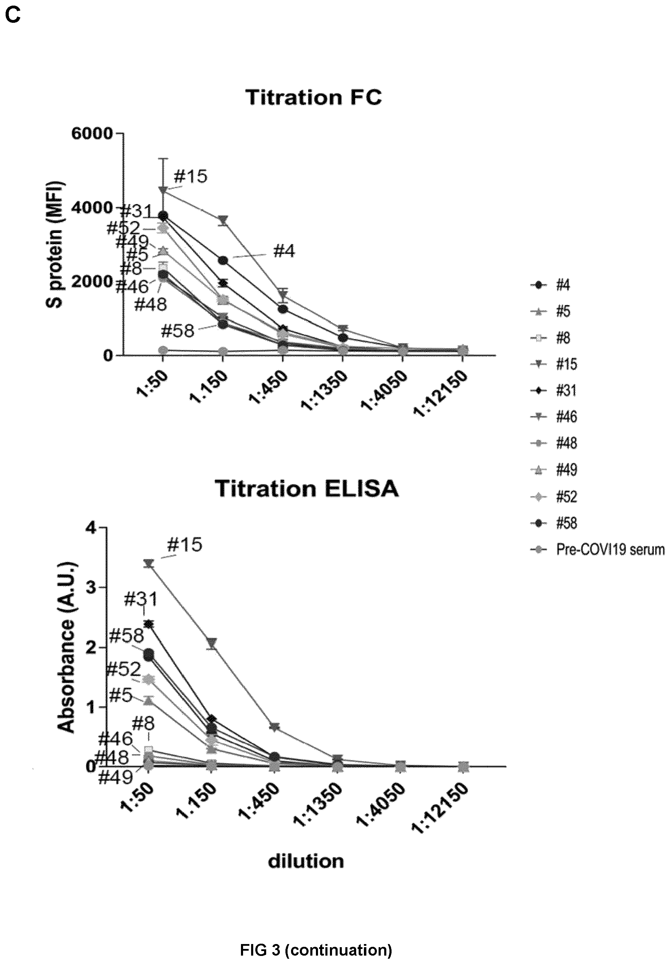

**Figure 3****. (a)** Box and whiskers plot of MFI and absorbance data in human sera according to their clinical classification of COVID-19 symptoms. Clinical classification was done according to the following parameters: Asymptomatic, no symptoms; Mild, 3 or more of the following symptoms: non-productive cough, hyperthermia, headache, odynophagia, dyspnea, asthenia , myalgia, ageusia, anosmia, cutaneous involvement; Moderate, 3 or more of the above symptoms plus gastrointestinal symptoms, or more than 3 of the above for 7 or more days; Moderate-Severe, 3 or more of the above symptoms plus pneumonia; Severe, pneumonia requiring hospitalization and intubation. **(b)** Comparison of MFI versus absorbance data generated by flow cytometry and ELISA for all human sera. A lineal regression curve was adjusted, with a 95% confidence interval, to all data with the R2 values indicated in the plots. Selected samples of outliers (#4, #8, #31, #49) as well as samples close to a diagonal (#36, #12, #52, #15) are indicated. **(c)** Titration of selected human sera by ELISA using the S1 protein and by flow cytometry with Jurkat-S cells. Samples with antibodies detected by flow cytometry and not by ELISA are indicated (#8, #46, #48, #49). Data represent the mean±SD of triplicates.

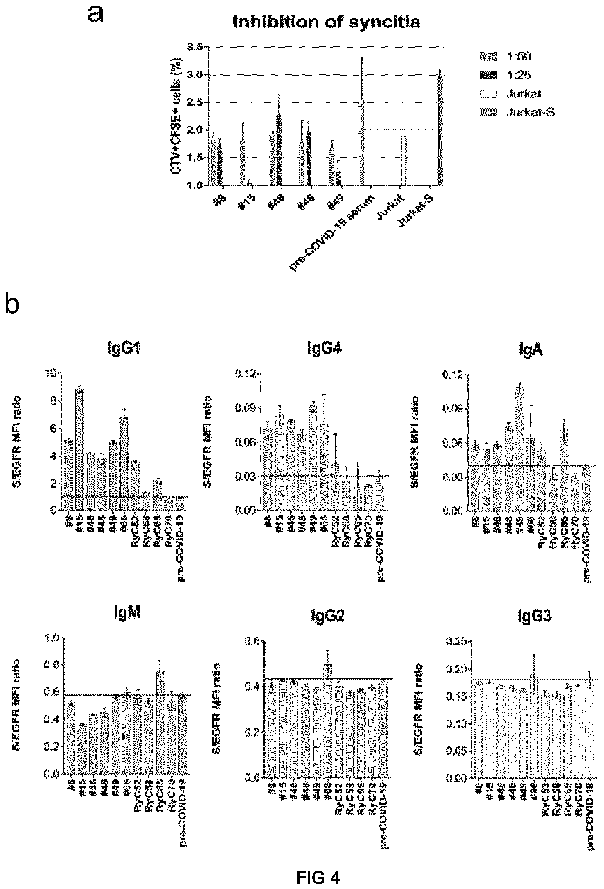

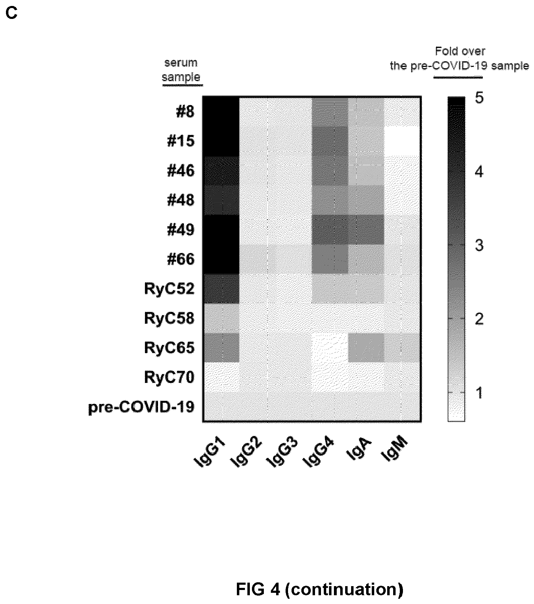

**Figure 4****. (a)** Inhibition of the formation of syncitia by sera from COVID-19 patients. CTV-labelled ACE2+ human cell line and the CFSE-labelled Jurkat-S cell line were incubated overnight in the presence of 1:50 or 1:25 dilutions of the indicated serum samples. A control of Jurkat-S cells incubated with HepG2 in the absence of sera and a control of parental Jurkat cells incubated with HepG2 were included. Data represent the mean±SD of triplicated datasets. **(b)** Detection of anti-S immunoglobulins of the indicated subclasses in a multiplexed method of flow cytometry in the serum samples indicated at 1:50 dilution. Data represent the mean±SD of triplicated datasets. **(c)** Heatmap plot of the anti-S protein antibody data shown in b, normalized as fold value of the S/egfr MFI ratio in each sample divided by the mean S/egfr MFI ratio of the pre-COVID-19 serum.

### EXAMPLES

### MATERIALS AND METHODS

### Cells

The human T-cell line Jurkat clone E6-1 was acquired from ATCC (TIB-152) were maintained in complete RPMI 1640 supplemented with 5% fetal bovine serum (FBS, Sigma) in a 5% CO₂ incubator. HEK293T cells were acquired from ATCC (CRL-3216) and maintained in DMEM supplemented with 10% FBS in a CO₂ incubator. Both cell lines were routinely tested for the absence of contaminating mycoplasma.

### Human sera

A total of 84 human sera were obtained from volunteers that contacted EMPIREO S.L. (www.empireo.es) for serological tests. Each participant provided written consent to participate in the study which was performed according to the EU guidelines and the Declaration of Helsinki. Serum donors filled in a questionnaire to allow their clinical classification according to the following parameters:
- Asymptomatic: no symptoms
- Mild: 3 or more of the following symptoms: non-productive cough, hyperthermia, headache, odynophagia, dyspnea, asthenia, myalgia, ageusia, anosmia, cutaneous involvement;
- Moderate: 3 or more of the above symptoms plus gastrointestinal symptoms, or more than 3 of the above for 7 or more days;
- Moderate-Severe: 3 or more of the above symptoms plus pneumonia;
- Severe: pneumonia requiring hospitalization and intubation.

### Lentiviral vector and Jurkat cell transduction

To express the full-length spike S protein of SARS-CoV2, the inventors used the lentiviral vector based on the epHIV-7 plasmid that contains the truncated version of human EGFR (huEGFRt) that lacks the domains essential for ligand binding and tyrosine kinase activity described in Wang, X. et al. A transgene-encoded cell surface polypeptide for selection, in vivo tracking, and ablation of engineered cells. Blood 118, 1255-1263 (2011*).* The vector version final comprising the nucleotide sequence SEQ ID NO: 4.

For transduction, lentiviral-transducing supernatants were produced from transfected packaging HEK-293T cells. Briefly, lentiviruses were obtained by co-transfecting plasmids psPAX2 (gag/pol) and using the JetPEI transfection reagent (Polyplus Transfection). Viral supernatants were obtained after 24 and 48 hours of transfection. Polybrene (8 µg/mL) was added to the viral supernatants prior to transduction of Jurkat cells. A total of 3x105 Jurkat cells were plated on a P24 flat-bottom well 350 µL of DMEM and 350 µL of viral supernatant were added. Cells were centrifuged for 90 minutes at 2200 rpm and left in culture for 24 hours. Transduced cells were selected by FACS sorting 48 h later using the anti-EGFR antibody.

### Flow cytometry

Jurkat-S cells were incubated for 30 min on ice with 1:50 dilutions of human sera in phosphate-buffered saline (PBS), 1% bovine serum albumin (BSA), 0.02% sodium azide. Cells were spun for 5 min at 900 g and the pellet was resuspended in PBS-BSA buffer and spun to eliminate the excess of antibody. Two additional washes were carried out. The cell pellet was finally resuspended in a 1:200 dilution of mouse anti-human IgG1 Fc-PE (Ref.: 9054-09, Southern Biotech) and a 1:300 dilution of the Brilliant Violet 421^{™} anti-human EGFR Antibody (Ref.: 352911, Biolegend) in PBS-BSA. Samples were then washed and labelled cells were analyzed on a FACSCalibur or FACSCanto II flow cytometer (Becton-Dickinson) and the data were analyzed with FlowJo software (TreeStar). Other antibodies were: BV605-labelled mouse anti-human IgM (Ref.: 562977, BD Biosciences); mouse anti-human IgG2 Fc-FITC (Ref.: 9060-02, Southern Biotech); mouse anti-human lgG3 Hinge-Alexa Fluor 647 (Ref.: 9210-31, Southern Biotech); mouse anti-human lgG4 Fc-BIOT (Ref.: 9200-08, Southern Biotech); PerCP AffiniPure goat anti-human serum IgA, α chain specific (Ref.: 109-125-011, Jackson ImmunoResearch).

### Commercial kits

The following commercial serological tests were assayed:
- Feal Test of Hangzhou Alltest Biotech, reference number RPP25COV1925.
- Sienna Test of Salofa Oy, reference number 102221.
- BiosSynex Test of Biosynex Swiss, reference number SW40005.
- Vircell ELISA test of Vircell SL, reference number G1032.
- COVID-19 VIRCLIA IgM+IgA monotest, Vircell SL, reference number VCM098.

### ELISA

96 well plates (MaxiSorp NUNC-Immunoplate) were coated overnight at 4°C with S1 (2 µg/ml) and were subsequently blocked for 1 h with 1% BSA (Sigma). Coated plates were incubated with the diluted sera for 1.5 h at room temperature. Bound antibodies were detected by incubation with mouse anti-human IgG1 secondary antibody coupled to horse-radish-peroxidase (HRP) (Southern Biotech) diluted 1/6,000 in 1% BSA in PBS which was then detected using an ABTS substrate solution (Invitrogen). The OD at 415 nm was determined on a iMark microplate reader (Bio-Rad). Serum from a healthy individual previous to COVID-19 was used as negative control.

### Immunoprecipitation and Western Blot

For surface biotinylation, 20 x 106 Jurkat-S cells were incubated for 45 min on ice with 0.5 mg/ml of sulfo-NHS-biotin (Pierce) in PBS supplemented with 0.1 mM CaCl₂ and 1 mM MgCl₂. After washing, the cells were lysed in Brij96 lysis buffer (0.33% Brij96, 150 mM NaCl; 20 mM Tris-HCI pH 7.8; 10 mM iodoacetamide; 1 mM PMSF; 1 µg/ml aprotinin; 1 µg/ml leupeptin) and pull-down was carried out Streptavidin-agarose beads. The bead-bound material was washed 5 times and subjected to SDS-PAGE. The proteins recovered were then transferred to a nitrocelullose membrane that was probed with with a mix of sera from donors #15, #3, #4, #8 and #66, diluted 1:2000. The nitrocellulose membrane was developed by ECL (Pierce). ECL autoradiography films were quantified with ImageJ Software.

### Neutralization assay with pseudotyped virus

Lentiviral supernatants were produced from transfected HEK-293T cells as described previously (*Martinez-Martin et al., 2009*). Briefly, lentiviruses were obtained by co-transfecting plasmids psPAX2 (gag/pol), pHRSIN-GFP and either HIV-1 envelope (pCMV-NL4.3) or VSV envelope (pMD2.G) using the JetPEI transfection reagent (Polyplus Transfection). Viral supernatants were obtained after 24 and 48 hours of transfection. Polybrene (8µg/mL) was added to the viral supernatants prior to transduction of Jurkat cells. A total of 3x105 MOLT-4 cells were plated on a P24 flat-bottom well 350 µL of DMEM and 350 µL of viral supernatant were added. Cells were centrifuged for 90 minutes at 2200 rpm and left in culture for 24 hours.

### Formation of syncitia

To analyze the formation of syncitia between Jurkat-S and HepG2 cells, HepG2 cells were detached from the culture plate by trypsinization and resuspended at a concentration of 3x106 cells/ml in PBS. Jurkat-S cells were collected by centrifugation and resuspended in PBS at the same concentration. HepG2 cells were labelled with Cell Trace Violet (CTV, Invitrogen) for 5 min at 37°C in PBS; Jurkat-S were labelled with CFDA-SE (CFSE, Invitrogen) in the same conditions. Both dyes were used at a final concentration of 5 µM. Labelling was stopped by adding complete medium and the cells washed with medium and finally mixed at different ratios in complete RPMI medium + 5% FBS and plated overnight at 37°C. Formation of syncitia was analyzed by flow cytometry by calculating the percentage of cells double positive for CFSE and CTV.

### RESULTS

The inventors have used a lentiviral vector to express the full spike "S" protein of SARS-CoV2 followed by a truncated human EGFR protein (huEGFRt) linked by a T2A self-cleaving sequence in transduced cells. This system allowed to express the two proteins from a monocystronic mRNA (Fig. 1a). They have produced transducing supernatants to express the construct in the human leukemic cell line Jurkat.

Cell surface biotinylation followed by immunoprecipitation with a mix of sera from recovered COVID-19 patients showed a polypeptide of 150 kD when resolve by SDS-PAGE under reducing conditions (Fig. 1b). Interestingly, the size of the polypeptide triplicated in size when samples were resolved in the absence of reducing agents under non-denaturing conditions (Fig. 1b). This data suggest that the S protein of SARS-CoV-2 is expressed on Jurkat-S plasma membrane as native trimers. To determine if the S protein expressed on Jurkat-S cells was functional, they analyzed if it could promote the formation of syncitia when Jurkat-S cells were co-incubated with the ACE2+ human hepatocarcinoma cell line HepG2. The inventors labelled Jurkat-S cells with the green dye CFSE and HepG2 with the violet dye Cell Trace Violet. After overnight incubation, they detected a Jurkat-S dose-dependent formation of mixed-cell syncitia that were not detected if the HepG2 were incubated with parental Jurkat cells not expressing the S protein (Fig. 1c). This data indicated that the S protein expressed in Jurkat-S cells has fusogenic activity and that it must be in a native conformation.

The Jurkat-S transduced cells were analyzed by flow cytometry using an anti-EGFR monoclonal antibody (mAb) to demonstrate the expression of the huEGFRt construct and with sera from SARS-CoV2-infected blood donors to demonstrate the expression of the S protein.

The Jurkat-S clone was stained with anti-EGFR mAb and either serum from a human blood donor extracted before the COVID-19 pandemic (pre-COVID-19 serum) or serum from an asymptomatic blood donor (donor #15) that was determined positive with several serological tests (Table 1). Jurkat-S cells were clearly double positive for EGFR receptor and for S protein when incubated with serum from donor #15 but only positive for EGFR when incubated with the pre-COVID-19 serum (Fig. 1d). The expression of S protein in the Jurkat-S clone allowed to detect anti-S protein in sera from individuals suspected to have been exposed to SARS-CoV2 virus as a serological test alternative to ELISA tests based on the expression of recombinant viral proteins.

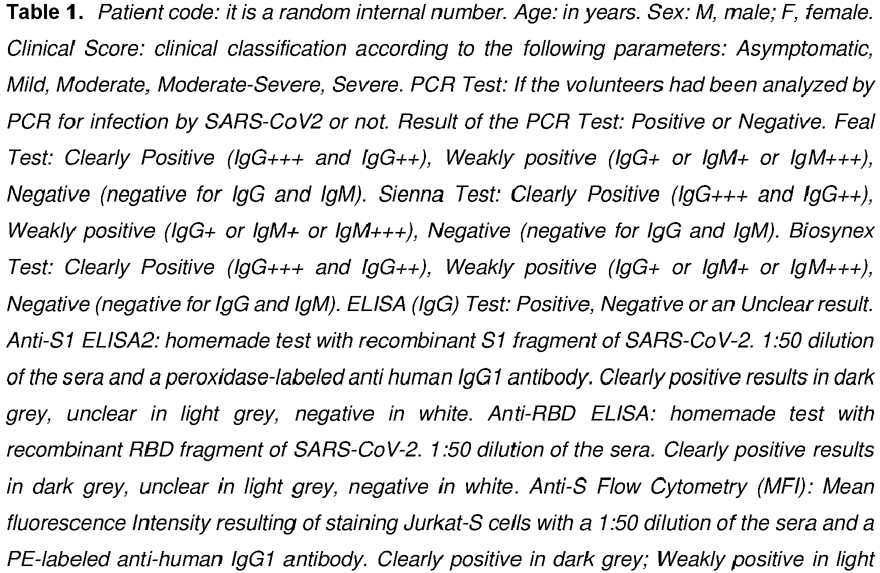

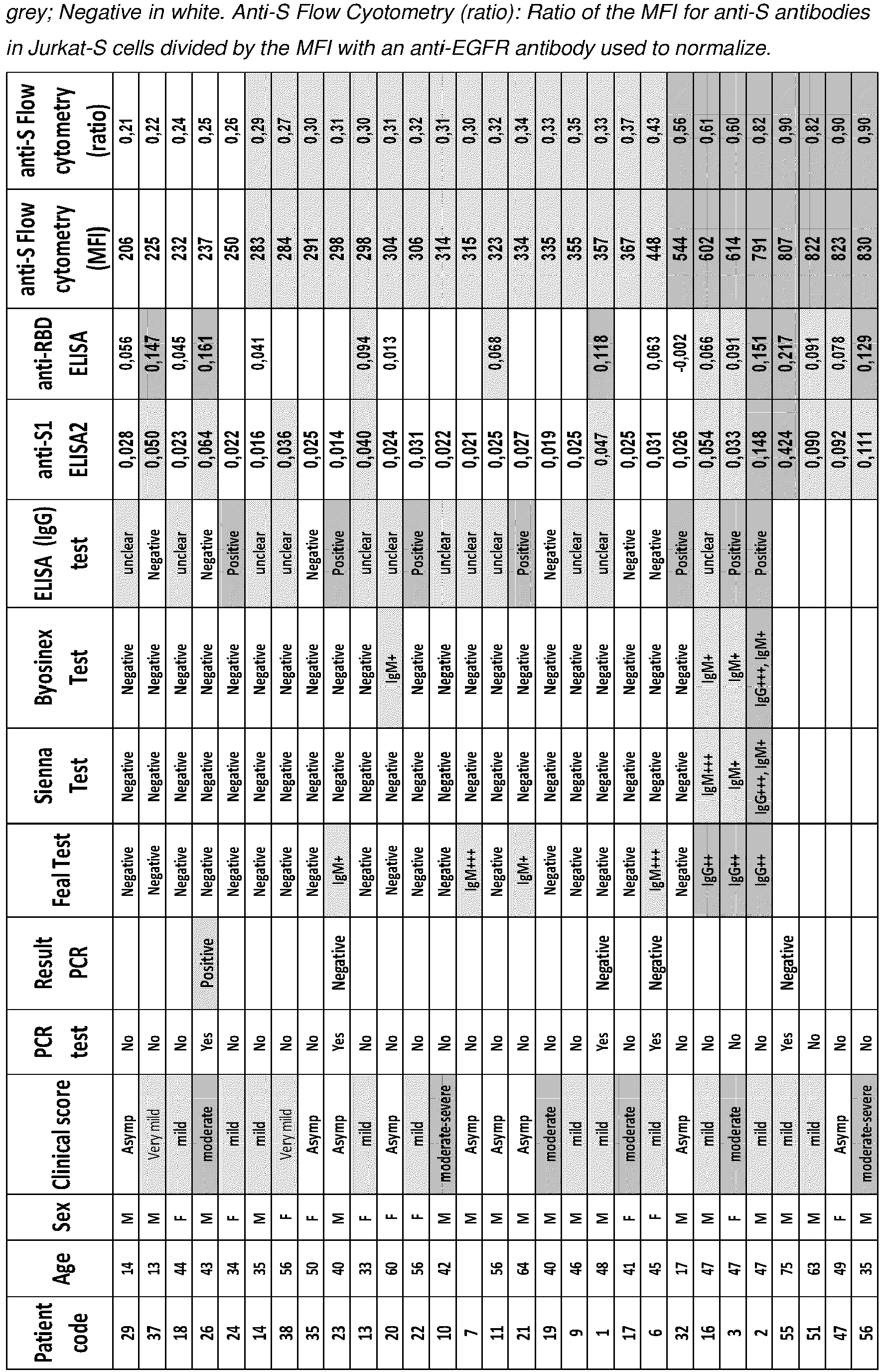

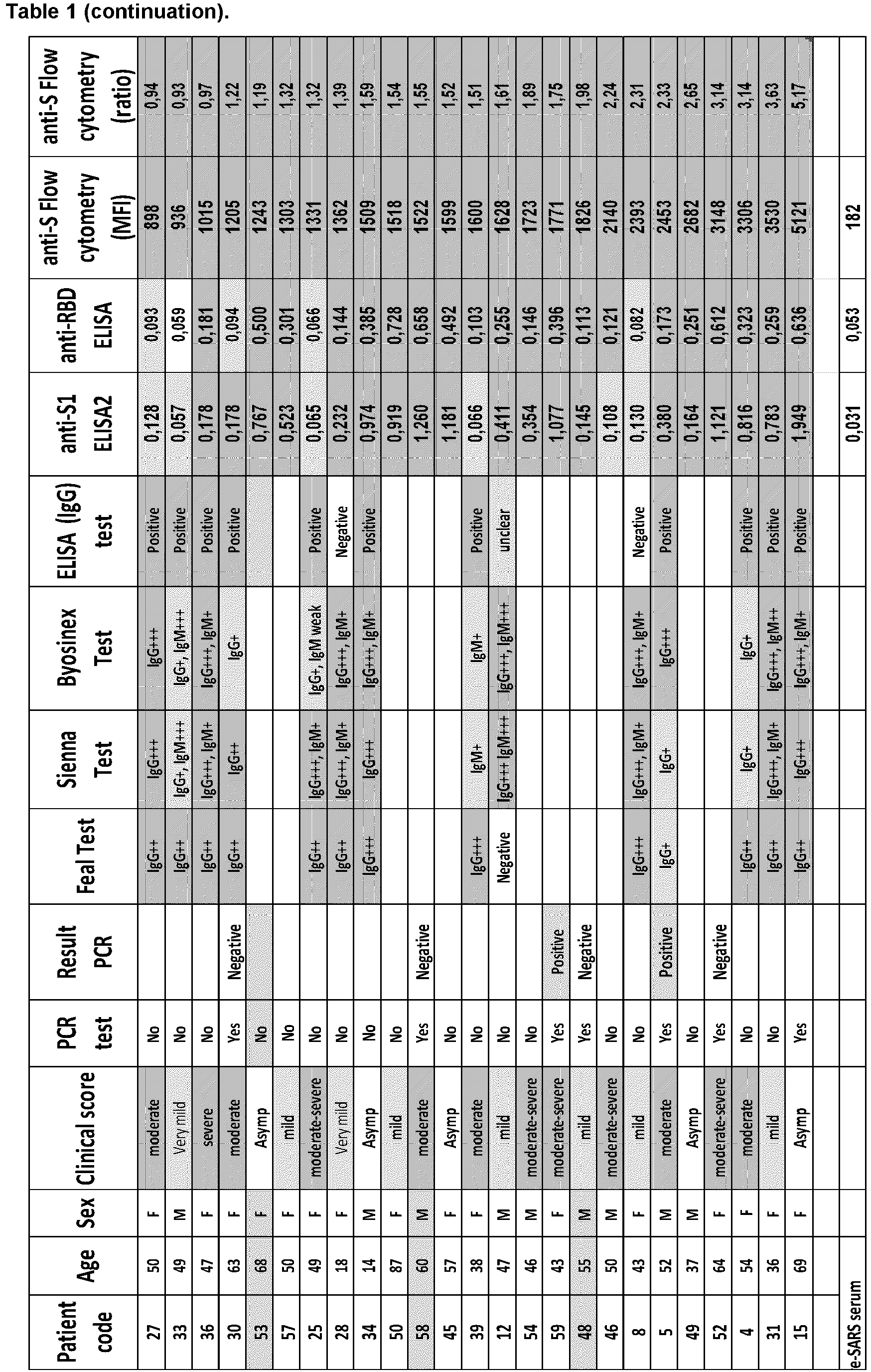

Indeed, incubating Jurkat-S cells with sera from different blood donors allows to detect a wide range of intensities of staining (Fig. 2a) that allows to generate numerical values (in terms of mean fluorescence intensity, MFI) that give a graded perspective of the existence of anti-total S protein antibodies in blood (Table 1). In comparison with commercial serological tests, the Jurkat-S clone-based flow cytometry (FC) test can detect more cases of seropositive individuals (Table 1). The wider dynamic range of the FC test with Jurkat-S was especially effective for those cases in which commercial test based on recombinant proteins gave uncertain results (Fig. 2b).

A special group of tested samples, corresponds to 30 health care personnel working at Hospital Ramon y Cajal of Madrid, who have been heavily exposed to infected patients during the March-May 2020 pandemic in Madrid, were tested for positivity by PCR. The PCR test in all those cases was always negative and the serological test carried out was also negative (Table 1). By contrast, the FC test with Jurkat-S showed that 2 of the sera (RyC52 and RyC65) were clearly positive for lgG1 anti-S antibody (Fig 2c). The S protein/EGFRt MFI ratio of a third serum (RyC58) was slightly above the threshold of 0.5 that have been defined to distinguish positive from negative sera. To discriminate samples that are near the threshold like that of RyC58, the inventors can plot the EFGR fluorescence intensity versus S protein binding fluorescence intensity as they did in Fig. 1d. Since the expression of S protein in Jurkat-S is coupled to the expression of EGFRt, a positive serum should produce a diagonal in the anti-S protein vs EGFR plot whereas a negative serum should generate a flat profile. In fact, this was the case for the borderline RyC58 serum and not for the RyC70 serum (Fig. 2d). Therefore, serum from donor RyC58 is clearly positive for lgG1 anti-S whereas serum from donor RyC70 is clearly negative.

In order to compare the FC test with ELISA tests, the inventors built a test using recombinant S1 fragment and recombinant RBD fragment of the S protein. The comparison of absorbance values in the ELISA tests with those of MFI in the FC test, shows a wider range of responses in all type of donors classified according to having experienced COVID-19 symptoms (asymptomatic, mild, moderate or moderate-severe to severe; Fig. 3a). Finally, the comparison of Absorbance values in the two ELISA tests (anti-S1 and anti-RBD) produced a perfect straight line, whereas the comparison of the FC MFI with the absorbance values (against S1 and RBD) does not adjust to a straight line (Fig. 3b). Indeed, there are clearly outlier serum samples that are more positive for FC than by ELISA, indicating that there can be many SARS-CoV2-exposed individuals who could be negative by ELISA in terms of expressing lgG1 against S recombinant protein fragments but result positive by FC. Those individuals could have antibodies against the total S protein that is exposed on the surface of the Jurkat-S clone that are not detected by ELISA. Sera from donors #8, #46, #48 and #49 were poorly detected by ELISA but easily detected by FC; sera from donors #15, #31 and #52 were clearly detected by both methods and serum from donor #58, appeared to be detected better by ELISA than by FC (Fig. 3b). To determine if those differences were maintained at different dilutions of the sera, a titration test was carried out in parallel using the FC Jurkat-S method and ELISA of the S1 fragment. The results showed that all sera, including that of donor #58, were clearly positive by FC even at a 1:450 dilution whereas by ELISA, sera #8, #46, #48 and #49 remained negative (Fig. 3c). These results indicate that sera which could have been considered negative by ELISA are indeed positive for lgG1 antibodies for S protein. Sample #49 was from an asymptomatic individual but sera #8, #46 and #48 were from individuals who had experienced from mild to severe symptoms (Table 1).

The inventors next tested if those sera that were detected by FC with Jurkat-S but not by ELISA had the capacity to neutralize the S protein. To this end, they used the syncitia formation assay between Jurkat-S and HepG2 cells shown in Fig. 1c. They found that sera #8, #15, #46, #48 and #49 were able to inhibit the formation of syncitia to levels of the negative control of syncitia formed by Jurkat that do not express the S protein (Fig. 4a). Finally, they tested if the Jurkat-S cells could be used in a multiplexed approach to test the expression of several human immunoglobulins anti-S in a single assay. They analyzed the presence of lgG1, lgG2, IgG3, IgG4, IgA and IgM antibodies able to bind Jurkat-S in several serum samples and calculated the S/egfr MFI ratio for each one of them (Table 2). The most robust binding was observed for lgG1 in all cases, followed by lgG4 and IgA (Fig. 4b). A weak binding of IgM was detected only in the RyC65 sample and IgG2 binding, although also weak, was detected in the #66 sample. IgG3 binding above background was not detected in any of the samples tested. A heatmap plot shows a summary of the data (Fig. 4c) and indicates that the predominant humoral response to the S protein in serum is in the form of IgG1, IgG4 and IgA.

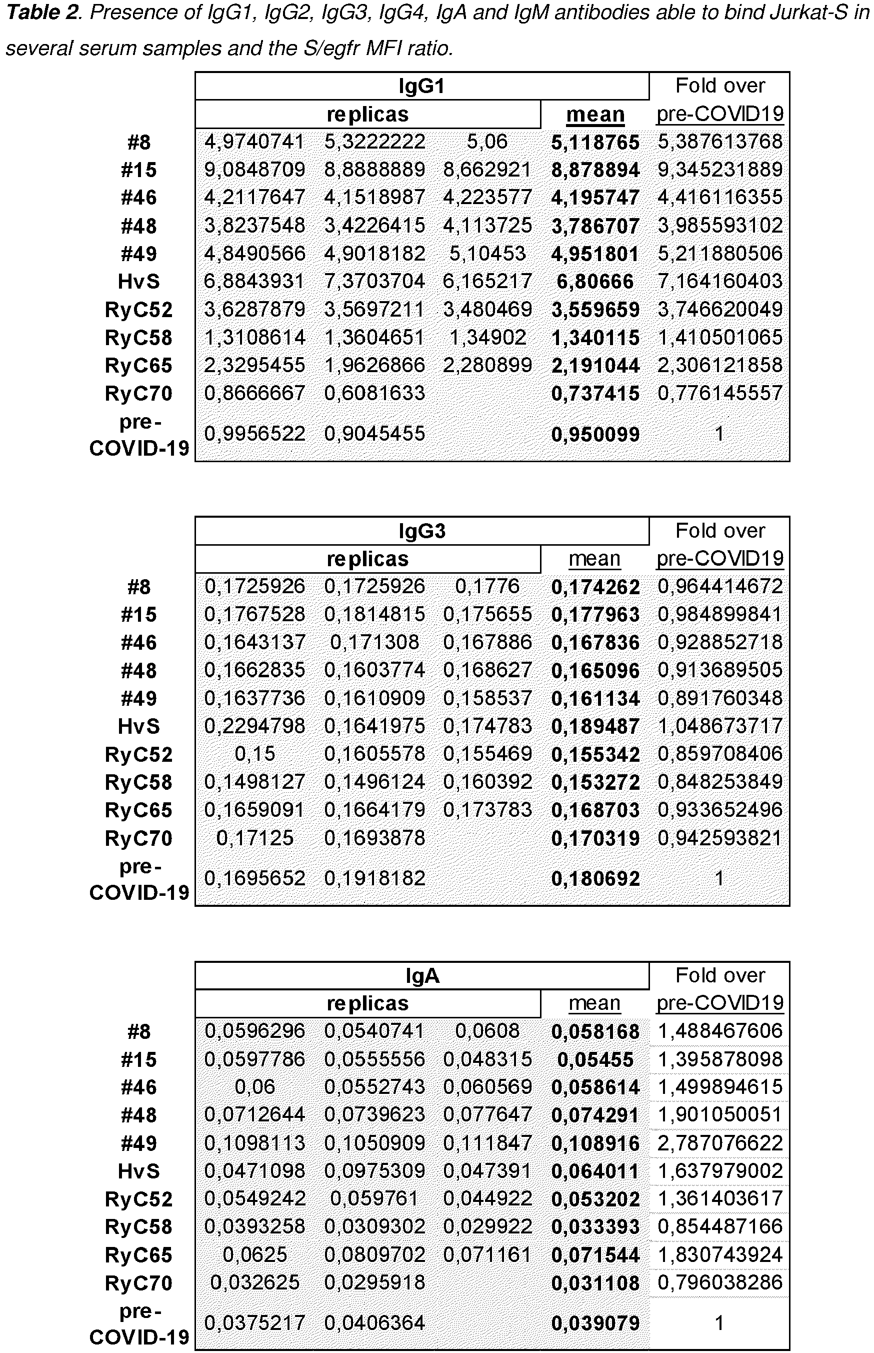

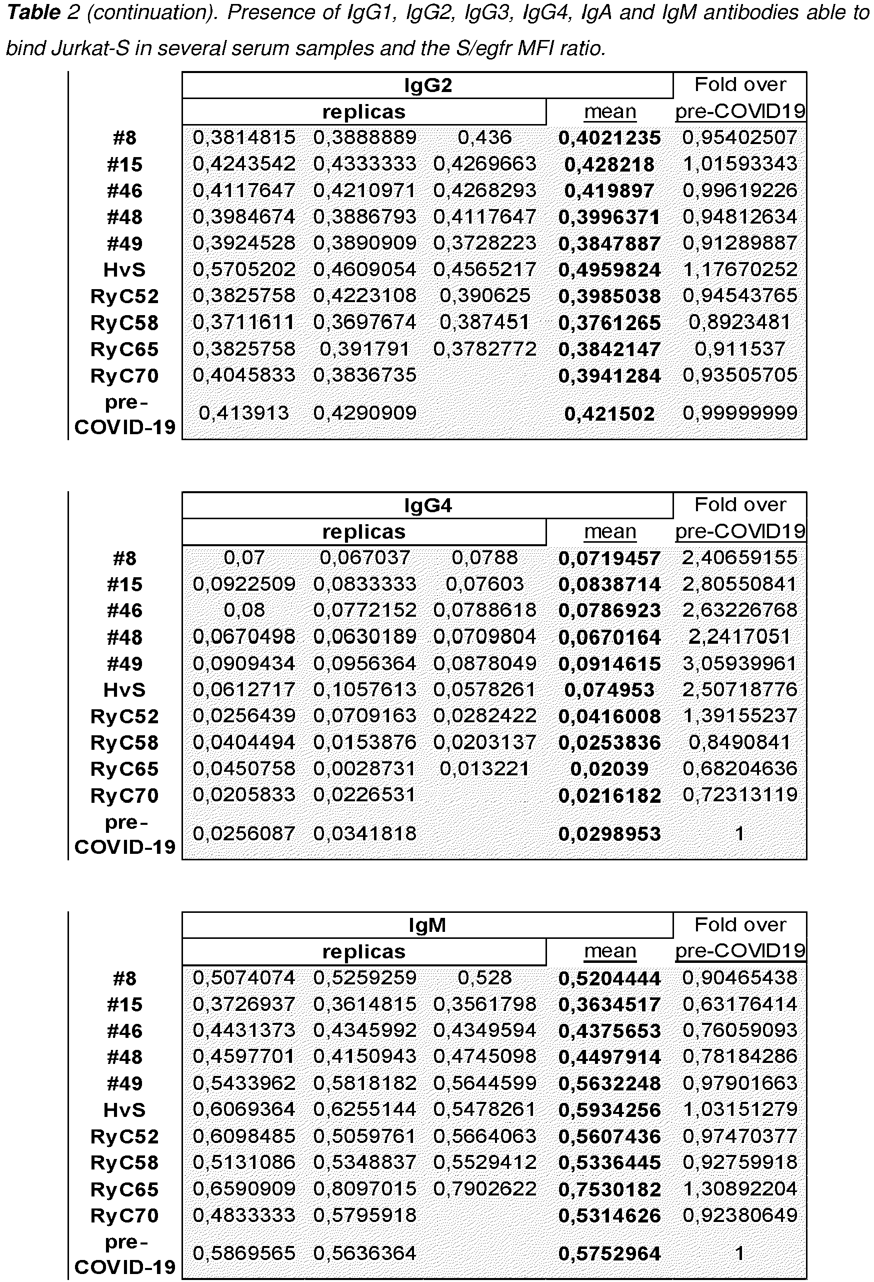

## Claims

1. A method for *in vitro* detection of anti-S protein antibodies against SARS-CoV2 by flow cytometry in a subject comprising the following steps:
(a) stable expressing either by transfection or viral transduction of a vector comprising a nucleotide sequence with at least 70% identity with the nucleotide sequence SEQ ID NO: 1 in a cell line in suspension,
(b) incubating the cell line obtained in step (a) with a sample isolated from the subject,
(c) incubating the sample obtained in step (b) with fluorescence labelled secondary antibodies, and
(d) measuring the mean fluorescence intensity of the sample obtained in step (c) by flow cytometry for detecting anti-S protein antibodies,
wherein mean fluorescence intensity of the sample is higher than a reference value, the subject is considered positive for anti-S protein antibodies.

2. A method for diagnostic of the exposure to SARS-CoV2 virus of a subject by flow cytometry comprising the following steps:
(a) stable expressing either by transfection or viral transduction of a vector comprising a nucleotide sequence with at least 70% identity with the nucleotide sequence SEQ ID NO: 1 in a cell line in suspension,
(b) incubating the cell line obtained in step (a) with a sample isolated from the subject,
(c) incubating the sample obtained in step (b) with fluorescence labelled secondary antibodies, and
(d) measuring the mean fluorescence intensity of the sample obtained in step (c) by flow cytometry for detecting anti-S protein antibodies,
wherein if mean fluorescence intensity of the sample is higher than a reference value, the subject has anti-S protein antibodies, and is indicative that the subject has been exposed to SARS-CoV2 virus.

3. The method according to claim 1 or 2 wherein the vector of step (a) further comprising a marker of transfection or viral transduction.

4. The method according to claim 3 wherein the marker is a truncated human EGFR protein, preferably separated to the nucleotide sequence with at least 70% identity with the nucleotide sequence SEQ ID NO: 1 by a proteolytic T2A cleavage site.

5. The method according to any of claims 1 to 4 wherein the cell line is a mammal cell line, preferably human cell, more preferably is a non-adherent hematopoietic human cell line.

6. The method according to claim 5 wherein the cell line in suspension is the human T cell leukemic line Jurkat.

7. The method according to any of claims 1 to 6 wherein the subject is a mammal, preferably a human.

8. The method according to any of claims 1 to 7 wherein the sample is selected from list consisting of: blood or mucosal fluid, preferably the sample is blood serum.

9. Kit or device for *in vitro* detection of anti-S protein antibodies against SARS-CoV2 or for diagnostic of the exposure to SARS-CoV2 virus in a sample isolated from a subject comprising:
(i) a cell line in suspension comprising a vector wherein the vector comprising a nucleotide sequence with at least 70% identity with the nucleotide sequence SEQ ID NO: 1,
(ii) fluorescence labelled secondary antibodies, and
(iii) means for carried out a detection by flow cytometry.

10. Kit or device according to claim 9 wherein the vector further comprising a marker of transfection or viral transduction.

11. Kit or device according to claim 10 wherein the marker is a truncated human EGFR protein, preferably separated to the nucleotide sequence with at least 70% identity with the nucleotide sequence SEQ ID NO: 1 by a proteolytic T2A cleavage site.

12. Kit or device according to any of the claims 9 to 11 wherein the cell line is a mammal cell line, preferably human cell, more preferably is a non-adherent hematopoietic human cell line.

13. Kit or device according to claim 12 wherein the cell line in suspension is the human T cell leukemic line Jurkat.

14. Use of the kit according to any of claims 9 to 13 for *in vitro* detection of antibodies against SARS-CoV2 in a sample isolated from a subject.

15. Use of the kit according to any of claims 9 to 13 for diagnostic of the exposure to SARS-CoV2 virus of a subject.

16. Use of the kit according to claims 14 or 15 wherein the subject is a mammal, preferably a human.

17. Use of the kit according to claims 14 to 16 wherein the sample is selected from list consisting of: blood or mucosal fluid, preferably the sample is blood serum.


SEQUENCE LISTING

<110>  Consejo Superior de Investigaciones Científicas (CSIC)

<120>  FLOW CYTOMETRY MULTIPLEXED METHOD FOR THE DETECTION OF SARS-CoV-2
        ANTIBODIES

<130>  EP1641.1588

<160>  4

<170>  PatentIn version 3.5

<210>  1
<211>  3780
<212>  DNA
<213>  Artificial Sequence

<220>
<223>  S protein of SARS-CoV2

<400>  1
cagtgtgtga acctgaccac cagaacacag ctgcctccag cctacaccaa cagctttacc      60

agaggcgtgt actaccccga caaggtgttc agatccagcg tgctgcactc tacccaggac     120

ctgttcctgc ctttcttcag caacgtgacc tggttccacg ccatccacgt gtccggcacc     180

aatggcacca agagattcga caaccccgtg ctgcccttca acgacggggt gtactttgcc     240

agcaccgaga agtccaacat catcagaggc tggatcttcg gcaccacact ggacagcaag     300

acccagagcc tgctgatcgt gaacaacgcc accaacgtgg tcatcaaagt gtgcgagttc     360

cagttctgca acgacccctt cctgggcgtc tactaccaca agaacaacaa gagctggatg     420

gaaagcgagt tccgggtgta cagcagcgcc aacaactgca ccttcgagta cgtgtcccag     480

cctttcctga tggacctgga aggcaagcag ggcaacttca agaacctgcg cgagttcgtg     540

ttcaagaaca tcgacggcta cttcaagatc tacagcaagc acacccctat caacctcgtg     600

cgggatctgc ctcagggctt ctctgctctg gaacccctgg tggatctgcc catcggcatc     660

aacatcaccc ggtttcagac actgctggcc ctgcacagaa gctacctgac acctggcgat     720

agcagcagcg gatggacagc tggtgccgcc gcttactatg tgggctacct gcagcctaga     780

accttcctgc tgaagtacaa cgagaacggc accatcaccg acgccgtgga ttgtgctctg     840

gatcctctga gcgagacaaa gtgcaccctg aagtccttca ccgtggaaaa gggcatctac     900

cagaccagca acttccgggt gcagcccacc gaatccatcg tgcggttccc caatatcacc     960

aatctgtgcc ccttcggcga ggtgttcaat gccaccagat tcgcctctgt gtacgcctgg    1020

aaccggaagc ggatcagcaa ttgcgtggcc gactactccg tgctgtacaa ctccgccagc    1080

ttcagcacct tcaagtgcta cggcgtgtcc cctaccaagc tgaacgacct gtgcttcaca    1140

aacgtgtacg ccgacagctt cgtgatccgg ggagatgaag tgcggcagat tgcccctgga    1200

cagacaggca agatcgccga ctacaactac aagctgcccg acgacttcac cggctgtgtg    1260

attgcctgga acagcaacaa cctggactcc aaagtcggcg gcaactacaa ttacctgtac 1320

cggctgttcc ggaagtccaa tctgaagccc ttcgagcggg acatctccac cgagatctat 1380

caggccggca gcaccccttg taacggcgtg gaaggcttca actgctactt cccactgcag 1440

tcctacggct ttcagcccac aaatggcgtg ggctatcagc cctacagagt ggtggtgctg 1500

agcttcgaac tgctgcatgc ccctgccaca gtgtgcggcc ctaagaaaag caccaatctc 1560

gtgaagaaca aatgcgtgaa cttcaacttc aacggcctga ccggcaccgg cgtgctgaca 1620

gagagcaaca agaagttcct gccattccag cagtttggcc gggatatcgc cgataccaca 1680

gacgccgtta gagatcccca gacactggaa atcctggaca tcaccccttg cagcttcggc 1740

ggagtgtctg tgatcacccc tggcaccaac accagcaatc aggtggcagt gctgtaccag 1800

gacgtgaact gtaccgaagt gcccgtggcc attcacgccg atcagctgac acctacatgg 1860

cgggtgtact ccaccggcag caatgtgttt cagaccagag ccggctgtct gatcggagcc 1920

gagcacgtga acaatagcta cgagtgcgac atccccatcg gcgctggcat ctgtgccagc 1980

taccagacac agacaaacag ccccagacgg gccagatctg tggccagcca gagcatcatt 2040

gcctacacaa tgtctctggg cgccgagaac agcgtggcct actccaacaa ctctatcgct 2100

atccccacca acttcaccat cagcgtgacc acagagatcc tgcctgtgtc catgaccaag 2160

accagcgtgg actgcaccat gtacatctgc ggcgattcca ccgagtgctc caacctgctg 2220

ctgcagtacg gcagcttctg cacccagctg aatagagccc tgacagggat cgccgtggaa 2280

caggacaaga acacccaaga ggtgttcgcc caagtgaagc agatctacaa gacccctcct 2340

atcaaggact tcggcggctt caatttcagc cagattctgc ccgatcctag caagcccagc 2400

aagcggagct tcatcgagga cctgctgttc aacaaagtga cactggccga cgccggcttc 2460

atcaagcagt atggcgattg tctgggcgac attgccgcca gggatctgat ttgcgcccag 2520

aagtttaacg gactgacagt gctgcctcct ctgctgaccg atgagatgat cgcccagtac 2580

acatctgccc tgctggccgg cacaatcaca agcggctgga catttggagc tggcgccgct 2640

ctgcagatcc cctttgctat gcagatggcc taccggttca acggcatcgg agtgacccag 2700

aatgtgctgt acgagaacca gaagctgatc gccaaccagt tcaacagcgc catcggcaag 2760

atccaggaca gcctgagcag cacagcaagc gccctgggaa agctgcagga cgtggtcaac 2820

cagaatgccc aggcactgaa caccctggtc aagcagctgt cctccaactt cggcgccatc 2880

agctctgtgc tgaacgatat cctgagcaga ctggacaagg tggaagccga ggtgcagatc 2940

gacagactga tcaccggaag gctgcagtcc ctgcagacct acgttaccca gcagctgatc 3000

agagccgccg agattagagc ctctgccaat ctggccgcca ccaagatgtc tgagtgtgtg 3060

ctgggccaga gcaagagagt ggacttttgc ggcaagggct accacctgat gagcttccct 3120

cagtctgccc ctcacggcgt ggtgtttctg cacgtgacat acgtgcccgc tcaagagaag 3180

aatttcacca ccgctccagc catctgccac gacggcaaag cccactttcc tagagaaggc 3240

gtgttcgtgt ccaacggcac ccattggttc gtgacccagc ggaacttcta cgagccccag 3300

atcatcacca ccgacaacac cttcgtgtct ggcaactgcg acgtcgtgat cggcattgtg 3360

aacaataccg tgtacgaccc tctgcagccc gagctggaca gcttcaaaga ggaactggat 3420

aagtacttta agaaccacac aagccccgac gtggacctgg gcgatatcag cggaatcaat 3480

gccagcgtcg tgaacatcca gaaagagatc gaccggctga acgaggtggc caagaatctg 3540

aacgagagcc tgatcgacct gcaagaactg gggaagtacg agcagtacat caagtggccc 3600

tggtacatct ggctgggctt tatcgccgga ctgattgcca tcgtgatggt cacaatcatg 3660

ctgtgttgca tgaccagctg ctgtagctgc ctgaagggct gttgtagctg tggcagctgc 3720

tgcaagttcg acgaggacga ttctgagccc gtgctgaagg gcgtgaaact gcactacacc 3780

<210> 2
<211> 1008
<212> DNA
<213> Artificial Sequence

<220>
<223> truncated human EGFR protein

<400> 2
cgcaaagtgt gtaacggaat aggtattggt gaatttaaag actcactctc cataaatgct 60

acgaatatta aacacttcaa aaactgcacc tccatcagtg gcgatctcca catcctgccg 120

gtggcattta ggggtgactc cttcacacat actcctcctc tggatccaca ggaactggat 180

attctgaaaa ccgtaaagga aatcacaggg tttttgctga ttcaggcttg gcctgaaaac 240

aggacggacc tccatgcctt tgagaaccta gaaatcatac gcggcaggac caagcaacat 300

ggtcagtttt ctcttgcagt cgtcagcctg aacataacat ccttgggatt acgctccctc 360

aaggagataa gtgatggaga tgtgataatt tcaggaaaca aaaatttgtg ctatgcaaat 420

acaataaact ggaaaaaact gtttgggacc tccggtcaga aaaccaaaat tataagcaac 480

agaggtgaaa acagctgcaa ggccacaggc caggtctgcc atgccttgtg ctcccccgag 540

ggctgctggg gcccggagcc cagggactgc gtctcttgcc ggaatgtcag ccgaggcagg 600

gaatgcgtgg acaagtgcaa ccttctggag ggtgagccaa gggagtttgt ggagaactct 660

gagtgcatac agtgccaccc agagtgcctg cctcaggcca tgaacatcac ctgcacagga 720

cggggaccag acaactgtat ccagtgtgcc cactacattg acggccccca ctgcgtcaag 780

acctgcccgg caggagtcat gggagaaaac aacaccctgg tctggaagta cgcagacgcc 840

ggccatgtgt gccacctgtg ccatccaaac tgcacctacg gatgcactgg gccaggtctt 900

gaaggctgtc caacgaatgg gcctaagatc ccgtccatcg ccactgggat ggtgggggcc 960

ctcctcttgc tgctggtggt ggccctgggg atcggcctct tcatgtga 1008

<210> 3
<211> 54
<212> DNA
<213> Artificial Sequence

<220>
<223> proteolytic T2A cleavage site

<400> 3
gagggcagag gaagtcttct aacatgcggt gacgtggagg agaatcccgg ccct 54

<210> 4
<211> 11845
<212> DNA
<213> Artificial Sequence

<220>
<223> Full sequence vector (S protein of SARS-CoV-2 -tEGFR-T2A)

<400> 4
   tgggtctctc tggttagacc agatctgagc ctgggagctc tctggctaac tagggaaccc 60
   actgcttaag cctcaataaa gcttgccttg agtgcttcaa gtagtgtgtg cccgtctgtt 120
   gtgtgactct ggtaactaga gatccctcag acccttttag tcagtgtgga aaatctctag 180
   cagtggcgcc cgaacaggga cttgaaagcg aaagggaaac cagaggagct ctctcgacgc 240
   aggactcggc ttgctgaagc gcgcacggca agaggcgagg ggcggcgact ggtgagtacg 300
   ccaaaaattt tgactagcgg aggctagaag gagagagatg ggtgcgagag cgtcagtatt 360
   aagcggggga gaattagatc gatgggaaaa aattcggtta aggccagggg gaaagaaaaa 420
   atataaatta aaacatatag tatgggcaag cagggagcta gaacgattcg cagttaatcc 480
   tggcctgtta gaaacatcag aaggctgtag acaaatactg ggacagctac aaccatccct 540
   tcagacagga tcagaagaac ttagatcatt atataataca gtagcaaccc tctattgtgt 600
   gcatcaaagg atagagataa aagacaccaa ggaagcttta gacaagatag aggaagagca 660
   aaacaaaagt aagaaaaaag cacagcaagc agcagctgac acaggacaca gcaatcaggt 720
   cagccaaaat taccctatag tgcagaacat ccaggggcaa atggtacatc aggccatatc 780
   acctagaact ttaaatgcat gggtaaaagt agtagaagag aaggctttca gcccagaagt 840
   gatacccatg ttttcagcat tatcagaagg agccacccca caagatttaa acaccatgct 900
   aaacacagtg gggggacatc aagcagccat gcaaatgtta aaagagacca tcaatgagga 960
   agctgcaggc aaagagaaga gtggtgcaga gagaaaaaag agcagtggga ataggagctt 1020
   tgttccttgg gttcttggga gcagcaggaa gcactatggg cgcagcgtca atgacgctga 1080
   cggtacaggc cagacaatta ttgtctggta tagtgcagca gcagaacaat ttgctgaggg 1140
   ctattgaggc gcaacagcat ctgttgcaac tcacagtctg gggcatcaag cagctccagg 1200
   caagaatcct ggctgtggaa agatacctaa aggatcaaca gctcctgggg atttggggtt 1260
   gctctggaaa actcatttgc accactgctg tgccttggat ctacaaatgg cagtattcat 1320
   ccacaatttt aaaagaaaag gggggattgg ggggtacagt gcaggggaaa gaatagtaga 1380
   cataatagca acagacatac aaactaaaga attacaaaaa caaattacaa aaattcaaaa 1440
   ttttcgggtt tattacaggg acagcagaga tccagtttgg ggatcaattg catgaagaat 1500
   ctgcttaggg ttaggcgttt tgcgctgctt cgcgaggatc tgcgatcgct ccggtgcccg 1560
   tcagtgggca gagcgcacat cgcccacagt ccccgagaag ttggggggag gggtcggcaa 1620
   ttgaaccggt gcctagagaa ggtggcgcgg ggtaaactgg gaaagtgatg tcgtgtactg 1680
   gctccgcctt tttcccgagg gtgggggaga accgtatata agtgcagtag tcgccgtgaa 1740
   cgttcttttt cgcaacgggt ttgccgccag aacacagctg aagcttcgag gggctcgcat 1800
   ctctccttca cgcgcccgcc gccctacctg aggccgccat ccacgccggt tgagtcgcgt 1860
   tctgccgcct cccgcctgtg gtgcctcctg aactgcgtcc gccgtctagg taagtttaaa 1920
   gctcaggtcg agaccgggcc tttgtccggc gctcccttgg agcctaccta gactcagccg 1980
   gctctccacg ctttgcctga ccctgcttgc tcaactctac gtctttgttt cgttttctgt 2040
   tctgcgccgt tacagatcca agctgtgacc ggcgcctacg gctagcgccg ccaccatgct 2100
   gctgctggtg accagcctgc tgctgtgcga gctgccccac cccgcctttc tgctgatccc 2160
   ccagtgtgtg aacctgacca ccagaacaca gctgcctcca gcctacacca acagctttac 2220
   cagaggcgtg tactaccccg acaaggtgtt cagatccagc gtgctgcact ctacccagga 2280
   cctgttcctg cctttcttca gcaacgtgac ctggttccac gccatccacg tgtccggcac 2340
   caatggcacc aagagattcg acaaccccgt gctgcccttc aacgacgggg tgtactttgc 2400
   cagcaccgag aagtccaaca tcatcagagg ctggatcttc ggcaccacac tggacagcaa 2460
   gacccagagc ctgctgatcg tgaacaacgc caccaacgtg gtcatcaaag tgtgcgagtt 2520
   ccagttctgc aacgacccct tcctgggcgt ctactaccac aagaacaaca agagctggat 2580
   ggaaagcgag ttccgggtgt acagcagcgc caacaactgc accttcgagt acgtgtccca 2640
   gcctttcctg atggacctgg aaggcaagca gggcaacttc aagaacctgc gcgagttcgt 2700
   gttcaagaac atcgacggct acttcaagat ctacagcaag cacaccccta tcaacctcgt 2760
   gcgggatctg cctcagggct tctctgctct ggaacccctg gtggatctgc ccatcggcat 2820
   caacatcacc cggtttcaga cactgctggc cctgcacaga agctacctga cacctggcga 2880
   tagcagcagc ggatggacag ctggtgccgc cgcttactat gtgggctacc tgcagcctag 2940
   aaccttcctg ctgaagtaca acgagaacgg caccatcacc gacgccgtgg attgtgctct 3000
   ggatcctctg agcgagacaa agtgcaccct gaagtccttc accgtggaaa agggcatcta 3060
   ccagaccagc aacttccggg tgcagcccac cgaatccatc gtgcggttcc ccaatatcac 3120
   caatctgtgc cccttcggcg aggtgttcaa tgccaccaga ttcgcctctg tgtacgcctg 3180
   gaaccggaag cggatcagca attgcgtggc cgactactcc gtgctgtaca actccgccag 3240
   cttcagcacc ttcaagtgct acggcgtgtc ccctaccaag ctgaacgacc tgtgcttcac 3300
   aaacgtgtac gccgacagct tcgtgatccg gggagatgaa gtgcggcaga ttgcccctgg 3360
   acagacaggc aagatcgccg actacaacta caagctgccc gacgacttca ccggctgtgt 3420
   gattgcctgg aacagcaaca acctggactc caaagtcggc ggcaactaca attacctgta 3480
   ccggctgttc cggaagtcca atctgaagcc cttcgagcgg gacatctcca ccgagatcta 3540
   tcaggccggc agcacccctt gtaacggcgt ggaaggcttc aactgctact tcccactgca 3600
   gtcctacggc tttcagccca caaatggcgt gggctatcag ccctacagag tggtggtgct 3660
   gagcttcgaa ctgctgcatg cccctgccac agtgtgcggc cctaagaaaa gcaccaatct 3720
   cgtgaagaac aaatgcgtga acttcaactt caacggcctg accggcaccg gcgtgctgac 3780
   agagagcaac aagaagttcc tgccattcca gcagtttggc cgggatatcg ccgataccac 3840
   agacgccgtt agagatcccc agacactgga aatcctggac atcacccctt gcagcttcgg 3900
   cggagtgtct gtgatcaccc ctggcaccaa caccagcaat caggtggcag tgctgtacca 3960
   ggacgtgaac tgtaccgaag tgcccgtggc cattcacgcc gatcagctga cacctacatg 4020
   gcgggtgtac tccaccggca gcaatgtgtt tcagaccaga gccggctgtc tgatcggagc 4080
   cgagcacgtg aacaatagct acgagtgcga catccccatc ggcgctggca tctgtgccag 4140
   ctaccagaca cagacaaaca gccccagacg ggccagatct gtggccagcc agagcatcat 4200
   tgcctacaca atgtctctgg gcgccgagaa cagcgtggcc tactccaaca actctatcgc 4260
   tatccccacc aacttcacca tcagcgtgac cacagagatc ctgcctgtgt ccatgaccaa 4320
   gaccagcgtg gactgcacca tgtacatctg cggcgattcc accgagtgct ccaacctgct 4380
   gctgcagtac ggcagcttct gcacccagct gaatagagcc ctgacaggga tcgccgtgga 4440
   acaggacaag aacacccaag aggtgttcgc ccaagtgaag cagatctaca agacccctcc 4500
   tatcaaggac ttcggcggct tcaatttcag ccagattctg cccgatccta gcaagcccag 4560
   caagcggagc ttcatcgagg acctgctgtt caacaaagtg acactggccg acgccggctt 4620
   catcaagcag tatggcgatt gtctgggcga cattgccgcc agggatctga tttgcgccca 4680
   gaagtttaac ggactgacag tgctgcctcc tctgctgacc gatgagatga tcgcccagta 4740
   cacatctgcc ctgctggccg gcacaatcac aagcggctgg acatttggag ctggcgccgc 4800
   tctgcagatc ccctttgcta tgcagatggc ctaccggttc aacggcatcg gagtgaccca 4860
   gaatgtgctg tacgagaacc agaagctgat cgccaaccag ttcaacagcg ccatcggcaa 4920
   gatccaggac agcctgagca gcacagcaag cgccctggga aagctgcagg acgtggtcaa 4980
   ccagaatgcc caggcactga acaccctggt caagcagctg tcctccaact tcggcgccat 5040
   cagctctgtg ctgaacgata tcctgagcag actggacaag gtggaagccg aggtgcagat 5100
   cgacagactg atcaccggaa ggctgcagtc cctgcagacc tacgttaccc agcagctgat 5160
   cagagccgcc gagattagag cctctgccaa tctggccgcc accaagatgt ctgagtgtgt 5220
   gctgggccag agcaagagag tggacttttg cggcaagggc taccacctga tgagcttccc 5280
   tcagtctgcc cctcacggcg tggtgtttct gcacgtgaca tacgtgcccg ctcaagagaa 5340
   gaatttcacc accgctccag ccatctgcca cgacggcaaa gcccactttc ctagagaagg 5400
   cgtgttcgtg tccaacggca cccattggtt cgtgacccag cggaacttct acgagcccca 5460
   gatcatcacc accgacaaca ccttcgtgtc tggcaactgc gacgtcgtga tcggcattgt 5520
   gaacaatacc gtgtacgacc ctctgcagcc cgagctggac agcttcaaag aggaactgga 5580
   taagtacttt aagaaccaca caagccccga cgtggacctg ggcgatatca gcggaatcaa 5640
   tgccagcgtc gtgaacatcc agaaagagat cgaccggctg aacgaggtgg ccaagaatct 5700
   gaacgagagc ctgatcgacc tgcaagaact ggggaagtac gagcagtaca tcaagtggcc 5760
   ctggtacatc tggctgggct ttatcgccgg actgattgcc atcgtgatgg tcacaatcat 5820
   gctgtgttgc atgaccagct gctgtagctg cctgaagggc tgttgtagct gtggcagctg 5880
   ctgcaagttc gacgaggacg attctgagcc cgtgctgaag ggcgtgaaac tgcactacac 5940
   cctcgagggc ggcggagagg gcagaggaag tcttctaaca tgcggtgacg tggaggagaa 6000
   tcccggccct aggatgcttc tcctggtgac aagccttctg ctctgtgagt taccacaccc 6060
   agcattcctc ctgatcccac gcaaagtgtg taacggaata ggtattggtg aatttaaaga 6120
   ctcactctcc ataaatgcta cgaatattaa acacttcaaa aactgcacct ccatcagtgg 6180
   cgatctccac atcctgccgg tggcatttag gggtgactcc ttcacacata ctcctcctct 6240
   ggatccacag gaactggata ttctgaaaac cgtaaaggaa atcacagggt ttttgctgat 6300
   tcaggcttgg cctgaaaaca ggacggacct ccatgccttt gagaacctag aaatcatacg 6360
   cggcaggacc aagcaacatg gtcagttttc tcttgcagtc gtcagcctga acataacatc 6420
   cttgggatta cgctccctca aggagataag tgatggagat gtgataattt caggaaacaa 6480
   aaatttgtgc tatgcaaata caataaactg gaaaaaactg tttgggacct ccggtcagaa 6540
   aaccaaaatt ataagcaaca gaggtgaaaa cagctgcaag gccacaggcc aggtctgcca 6600
   tgccttgtgc tcccccgagg gctgctgggg cccggagccc agggactgcg tctcttgccg 6660
   gaatgtcagc cgaggcaggg aatgcgtgga caagtgcaac cttctggagg gtgagccaag 6720
   ggagtttgtg gagaactctg agtgcataca gtgccaccca gagtgcctgc ctcaggccat 6780
   gaacatcacc tgcacaggac ggggaccaga caactgtatc cagtgtgccc actacattga 6840
   cggcccccac tgcgtcaaga cctgcccggc aggagtcatg ggagaaaaca acaccctggt 6900
   ctggaagtac gcagacgccg gccatgtgtg ccacctgtgc catccaaact gcacctacgg 6960
   atgcactggg ccaggtcttg aaggctgtcc aacgaatggg cctaagatcc cgtccatcgc 7020
   cactgggatg gtgggggccc tcctcttgct gctggtggtg gccctgggga tcggcctctt 7080
   catgtgagcg gccgctctag acccgggctg caggaattcg atatcaagct tatcgataat 7140
   caacctctgg attacaaaat ttgtgaaaga ttgactggta ttcttaacta tgttgctcct 7200
   tttacgctat gtggatacgc tgctttaatg cctttgtatc atgctattgc ttcccgtatg 7260
   gctttcattt tctcctcctt gtataaatcc tggttgctgt ctctttatga ggagttgtgg 7320
   cccgttgtca ggcaacgtgg cgtggtgtgc actgtgtttg ctgacgcaac ccccactggt 7380
   tggggcattg ccaccacctg tcagctcctt tccgggactt tcgctttccc cctccctatt 7440
   gccacggcgg aactcatcgc cgcctgcctt gcccgctgct ggacaggggc tcggctgttg 7500
   ggcactgaca attccgtggt gttgtcgggg aaatcatcgt cctttccttg gctgctcgcc 7560
   tgtgttgcca cctggattct gcgcgggacg tccttctgct acgtcccttc ggccctcaat 7620
   ccagcggacc ttccttcccg cggcctgctg ccggctctgc ggcctcttcc gcgtcttcgc 7680
   cttcgccctc agacgagtcg gatctccctt tgggccgcct ccccgcatcg ataccgtcga 7740
   ctagccgtac ctttaagacc aatgacttac aaggcagctg tagatcttag ccacttttta 7800
   aaagaaaagg ggggactgga agggctaatt cactcccaaa gaagacaaga tctgcttttt 7860
   gcctgtactg ggtctctctg gttagaccag atctgagcct gggagctctc tggctaacta 7920
   gggaacccac tgcttaagcc tcaataaagc ttgccttgag tgcttcaagt agtgtgtgcc 7980
   cgtctgttgt gtgactctgg taactagaga tccctcagac ccttttagtc agtgtggaaa 8040
   atctctagca gaattcgata tcaagcttat cgataccgtc gacctcgagg gggggcccgg 8100
   tacccaattc gccctatagt gagtcgtatt acaattcact ggccgtcgtt ttacaacgtc 8160
   gtgactggga aaaccctggc gttacccaac ttaatcgcct tgcagcacat ccccctttcg 8220
   ccagctggcg taatagcgaa gaggcccgca ccgatcgccc ttcccaacag ttgcgcagcc 8280
   tgaatggcga atggaaattg taagcgttaa tattttgtta aaattcgcgt taaatttttg 8340
   ttaaatcagc tcatttttta accaataggc cgaaatcggc aaaatccctt ataaatcaaa 8400
   agaatagacc gagatagggt tgagtgttgt tccagtttgg aacaagagtc cactattaaa 8460
   gaacgtggac tccaacgtca aagggcgaaa aaccgtctat cagggcgatg gcccactacg 8520
   tgaaccatca ccctaatcaa gttttttggg gtcgaggtgc cgtaaagcac taaatcggaa 8580
   ccctaaaggg agcccccgat ttagagcttg acggggaaag ccggcgaacg tggcgagaaa 8640
   ggaagggaag aaagcgaaag gagcgggcgc tagggcgctg gcaagtgtag cggtcacgct 8700
   gcgcgtaacc accacacccg ccgcgcttaa tgcgccgcta cagggcgcgt caggtggcac 8760
   ttttcgggga aatgtgcgcg gaacccctat ttgtttattt ttctaaatac attcaaatat 8820
   gtatccgctc atgagacaat aaccctgata aatgcttcaa taatattgaa aaaggaagag 8880
   tatgagtatt caacatttcc gtgtcgccct tattcccttt tttgcggcat tttgccttcc 8940
   tgtttttgct cacccagaaa cgctggtgaa agtaaaagat gctgaagatc agttgggtgc 9000
   acgagtgggt tacatcgaac tggatctcaa cagcggtaag atccttgaga gttttcgccc 9060
   cgaagaacgt tttccaatga tgagcacttt taaagttctg ctatgtggcg cggtattatc 9120
   ccgtattgac gccgggcaag agcaactcgg tcgccgcata cactattctc agaatgactt 9180
   ggttgagtac tcaccagtca cagaaaagca tcttacggat ggcatgacag taagagaatt 9240
   atgcagtgct gccataacca tgagtgataa cactgcggcc aacttacttc tgacaacgat 9300
   cggaggaccg aaggagctaa ccgctttttt gcacaacatg ggggatcatg taactcgcct 9360
   tgatcgttgg gaaccggagc tgaatgaagc cataccaaac gacgagcgtg acaccacgat 9420
   gcctgtagca atggcaacaa cgttgcgcaa actattaact ggcgaactac ttactctagc 9480
   ttcccggcaa caattaatag actggatgga ggcggataaa gttgcaggac cacttctgcg 9540
   ctcggccctt ccggctggct ggtttattgc tgataaatct ggagccggtg agcgtgggtc 9600
   tcgcggtatc attgcagcac tggggccaga tggtaagccc tcccgtatcg tagttatcta 9660
   cacgacgggg agtcaggcaa ctatggatga acgaaataga cagatcgctg agataggtgc 9720
   ctcactgatt aagcattggt aactgtcaga ccaagtttac tcatatatac tttagattga 9780
   tttaaaactt catttttaat ttaaaaggat ctaggtgaag atcctttttg ataatctcat 9840
   gaccaaaatc ccttaacgtg agttttcgtt ccactgagcg tcagaccccg tagaaaagat 9900
   caaaggatct tcttgagatc ctttttttct gcgcgtaatc tgctgcttgc aaacaaaaaa 9960
   accaccgcta ccagcggtgg tttgtttgcc ggatcaagag ctaccaactc tttttccgaa 10020
   ggtaactggc ttcagcagag cgcagatacc aaatactgtt cttctagtgt agccgtagtt 10080
   aggccaccac ttcaagaact ctgtagcacc gcctacatac ctcgctctgc taatcctgtt 10140
   accagtggct gctgccagtg gcgataagtc gtgtcttacc gggttggact caagacgata 10200
   gttaccggat aaggcgcagc ggtcgggctg aacggggggt tcgtgcacac agcccagctt 10260
   ggagcgaacg acctacaccg aactgagata cctacagcgt gagctatgag aaagcgccac 10320
   gcttcccgaa gggagaaagg cggacaggta tccggtaagc ggcagggtcg gaacaggaga 10380
   gcgcacgagg gagcttccag ggggaaacgc ctggtatctt tatagtcctg tcgggtttcg 10440
   ccacctctga cttgagcgtc gatttttgtg atgctcgtca ggggggcgga gcctatggaa 10500
   aaacgccagc aacgcggcct ttttacggtt cctggccttt tgctggcctt ttgctcacat 10560
   gttctttcct gcgttatccc ctgattctgt ggataaccgt attaccgcct ttgagtgagc 10620
   tgataccgct cgccgcagcc gaacgaccga gcgcagcgag tcagtgagcg aggaagcgga 10680
   agagcgccca atacgcaaac cgcctctccc cgcgcgttgg ccgattcatt aatgcagctg 10740
   gcacgacagg tttcccgact ggaaagcggg cagtgagcgc aacgcaatta atgtgagtta 10800
   gctcactcat taggcacccc aggctttaca ctttatgctt ccggctcgta tgttgtgtgg 10860
   aattgtgagc ggataacaat ttcacacagg aaacagctat gaccatgatt acgccaagct 10920
   cgaaattaac cctcactaaa gggaacaaaa gctggagctc caccgcggtg gcggcctcga 10980
   ggtcgagatc cggtcgacca gcaaccatag tcccgcccct aactccgccc atcccgcccc 11040
   taactccgcc cagttccgcc cattctccgc cccatggctg actaattttt tttatttatg 11100
   cagaggccga ggccgcctcg gcctctgagc tattccagaa gtagtgagga ggcttttttg 11160
   gaggcctagg cttttgcaaa aagcttcgac ggtatcgatt ggctcatgtc caacattacc 11220
   gccatgttga cattgattat tgactagtta ttaatagtaa tcaattacgg ggtcattagt 11280
   tcatagccca tatatggagt tccgcgttac ataacttacg gtaaatggcc cgcctggctg 11340
   accgcccaac gacccccgcc cattgacgtc aataatgacg tatgttccca tagtaacgcc 11400
   aatagggact ttccattgac gtcaatgggt ggagtattta cggtaaactg cccacttggc 11460
   agtacatcaa gtgtatcata tgccaagtac gccccctatt gacgtcaatg acggtaaatg 11520
   gcccgcctgg cattatgccc agtacatgac cttatgggac tttcctactt ggcagtacat 11580
   ctacgtatta gtcatcgcta ttaccatggt gatgcggttt tggcagtaca tcaatgggcg 11640
   tggatagcgg tttgactcac ggggatttcc aagtctccac cccattgacg tcaatgggag 11700
   tttgttttgg caccaaaatc aacgggactt tccaaaatgt cgtaacaact ccgccccatt 11760
   gacgcaaatg ggcggtaggc gtgtacggaa ttcggagtgg cgagccctca gatcctgcat 11820
   ataagcagct gctttttgcc tgtac 11845